(19) **Europäisches Patentamt / European Patent Office / Office européen des brevets**

(11) **EP 3 858 866 A1**

(12) **EUROPEAN PATENT APPLICATION**
published in accordance with Art. 153(4) EPC

(43) Date of publication:
**04.08.2021 Bulletin 2021/31**

(21) Application number: **19866716.4**

(22) Date of filing: **25.09.2019**

(51) Int Cl.:
*C07K 19/00* (2006.01)   *C12P 21/02* (2006.01)
*A61K 38/26* (2006.01)   *A61P 3/10* (2006.01)
*A61P 3/04* (2006.01)

(86) International application number:
**PCT/CN2019/107709**

(87) International publication number:
**WO 2020/063628 (02.04.2020 Gazette 2020/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.09.2018   CN 201811125762**

(71) Applicant: **Jiangsu Gensciences Inc.**
**Nantong, Jiangsu 226000 (CN)**

(72) Inventors:
• **WANG, Yali**
  **Beijing 100176 (CN)**
• **CHEN, Xian**
  **Beijing 100176 (CN)**
• **ZHU, Luyan**
  **Beijing 100176 (CN)**
• **LIU, Bin**
  **Beijing 100176 (CN)**
• **WANG, Xiaoshan**
  **Beijing 100176 (CN)**
• **REN, Zijia**
  **Beijing 100176 (CN)**

• **ZHOU, Tingting**
  **Beijing 100176 (CN)**
• **YAN, Haixia**
  **Beijing 100176 (CN)**
• **XU, Yingying**
  **Beijing 100176 (CN)**
• **GAO, Huihui**
  **Beijing 100176 (CN)**
• **WANG, Jin**
  **Beijing 100176 (CN)**
• **XU, Yang**
  **Beijing 100176 (CN)**
• **LIU, Yahui**
  **Beijing 100176 (CN)**
• **MO, Weichuan**
  **Beijing 100176 (CN)**
• **CHEN, Xin**
  **Beijing 100176 (CN)**
• **GAO, Jie**
  **Beijing 100176 (CN)**
• **SU, Hongsheng**
  **Beijing 100176 (CN)**

(74) Representative: **Studio Torta S.p.A.**
**Via Viotti, 9**
**10121 Torino (IT)**

(54) **GLP1-FC FUSION PROTEIN AND CONJUGATE THEREOF**

(57)     Provided by the present invention are a novel GLP-1 fusion protein and a conjugate thereof, a pharmaceutical composition containing same, and a use thereof in reducing blood sugar or body weight, especially for treating diabetes, in particular type II diabetes.

FIG. 1

EP 3 858 866 A1

**Description**

**CROSS REFERENCE TO RELATED APPLICATIONS**

This application claims the priority of Chinese Patent Application No. 201811125762.9, filed on September 26, 2018, and titled with "GLP1-FC FUSION PROTEIN AND CONJUGATE THEREOF".

**FIELD**

The present invention relates to biologically active polypeptide fusion proteins and their polymer conjugates having long half-life *in vivo,* especially GLP-1 receptor agonists having long half-life *in vivo,* pharmaceutical compositions containing the same, and uses thereof in reducing blood glucose (especially in the treatment of diabetes, in particular type II diabetes) and controlling body weight.

**BACKGROUND**

With the development of economy, the extension of the average life of the population and the change of lifestyle, diabetes has become a major health care problem worldwide. Whether in developed or developing countries, the incidence of diabetes is rising sharply. In 2007, there were approximately 246 million diabetic patients globally, and one diabetic patient died every 10 seconds. It is estimated that by 2025, the number of global diabetic patients will reach 333 million. China has become the "epicenter" for diabetes with 40 million diabetic patients currently and about 5% diabetes incidence, and is the second largest country in the world for diabetes (the first is India). There are two types of diabetes, insulin-dependent diabetes (type I diabetes) and non-insulin-dependent diabetes (type II diabetes), where type II diabetes accounts for more than 90% of diabetic patients. Type II diabetes feature as dysregulation of insulin secretion or effect and β-cell dysfunction, which lead to metabolic disorder of fat, carbohydrate and protein, resulting in chronic hyperglycemia, and ultimately causing various microvascular, macrovascular and various organ complications. Nowadays, there are two types of drugs to control diabetes: 1) insulin secretion-stimulating drugs, such as sulfonylureas, meglitinides, dipeptidyl peptidase inhibitors, and GLP-1 analogues; and 2) non-insulin secretion-stimulating drugs, such as insulin, α-glucosidase inhibitors, biguanides, thiazolidinediones, insulin analogues, etc. At present, many traditional diabetes-treating drugs used clinically are powerless for patients with type II diabetes. Those drugs fail to suppress the progressive deterioration of islet β-cells, to reduce the level of glycosylated hemoglobin (HbA1c) in blood, or to prevent complications of diabetes, such as heart disease and kidney failure, and those drugs accompanied by varying degrees of side effects. Therefore, it is necessary to develop a novel drug for the treatment of type II diabetes.

Gut hormone glucagon-like peptide-1 (GLP-1) was identified in 1985, which is expressed by the proglucagon gene after eating and mainly secreted by intestinal mucosa L-cells. GLP-1 can stimulate islet β-cells to secrete insulin (J Med Chem, 47, 4128-4134, 2004) and play an important role in stabilizing blood glucose levels. Exogenous administration of GLP-1 can normalize blood glucose level in patients with type II diabetes (Diabetes Care, 15, 270-276, 1992; Lancet, 359, 824-830, 2002; Endoer. Rev, 16, 390-410, 1996; and Diabetologia, 28, 565-573, 1985). GLP-1 has the following functions: acting on islet β-cells in a glucose-dependent manner, promoting the transcription of insulin gene, increasing the biosynthesis and secretion of insulin, stimulating the proliferation and differentiation of β-cells, inhibiting β-cell apoptosis and thereby increasing the number of islet β-cells, inhibiting the secretion of glucagon, increasing the sensitivity of insulin receptors of peripheral cells, reducing HbA1c, suppressing appetite and feeding, and delaying gastric emptying (Diabetic Med, 18, 144-149, 2001; Diabetes, 51, 1443-1452, 2002; Diabetologia, 45, 1263-1273, 2002; Diabetes, 50, 525-529, 2001; Diabetes, 50, 725, 2001; Diabetes, 52, 365-371, 2003; Recent Prog. Hormne Res. 56, 377-399, 2001; Disbetologia, 39, 1546-1553, 1996; Am. J. Physicl Endocrinol. Metab, 281, E242-247, 2001; US patent 477967, 478017, 478425; Diabetes Care, 22, 403-408, 1999; J. Clin. Endocrinology and Metabolism, 88, 3082-3089, 2003; and Diabetes, 44, 1295, 1995). However, GLP-1 is easily degraded by dipeptidyl peptidase (DPPIV) *in vivo* with a half-life of less than 2 minutes, and thus can hardly become an effective anti-diabetic drug.

Due to the short half-life and poor physical and chemical stability, peptide drugs are easily degraded by various proteases *in vivo* so that they usually require multiple injections within a day, which brings physical, mental and financial burdens to the patients, and limits their drug compliance. Therefore, there is an urgent need in the art for drugs with new structures to extend their plasma cycles and increase their systemic drug exposure.

**SUMMARY**

After years of research and long-term experiments, the inventors have found that, after fusing a polypeptide with a fusion partner (e.g., Fc fragment, albumin, XTEN or transferrin) that is capable of extending the half-life of the polypeptide to generate a fusion protein, and further conjugating with a hydrophilic polymer (such as polyalkylene glycol, specifically PEG), this can effectively increase the *in vivo* stability of the biologically active polypeptides and improve the efficacy of drugs. The present invention was completed based on the above finding.

In the first aspect, the present invention provides a fusion protein of a GLP-1 receptor agonist, comprising a GLP-1 receptor agonist and a fusion partner (FP) capable of extending the half-life of the fusion protein *in vivo,* wherein the GLP-1 receptor agonist and the fusion partner are linked directly or through a first linker L1.

Preferably, the first linker L1 is a peptide containing 1, 2, 3, 4, 5, 6, 7, 8, 9, 10, or more amino acids;

More preferably, the first linker L1 is a flexible peptide containing A, T, G, and/or S, such as $(GS)_n$, where n is an integer from 1 to 50, for example, 1, 2, 3, 4, 5, 6, 7, 8, 9, or 10.

Most preferably, the first linker L1 is selected from the group consisting of:

GSGGGSGGGGSGGGGS (SEQ ID NO: 9),
GSGGGGSGGGGSGGGGSGGGGSGGGGS (SEQ ID NO: 10),
GGGGSGGGGSGGGGSGGGGS (SEQ ID NO: 11),
GSGGGGSGGGGSGGGGSGGGGSGGGGSGGGGSGGGGS (SEQ ID NO: 12),
GGGSGGGSGGGSGGGSGGGS (SEQ ID NO: 13),
PRFQDSSSSKAPPPSLPSPSRLPGPSDTPILPQ (SEQ ID NO: 14),
SSSSKAPPPSLPSPSRLPGPSDTPILPQ (SEQ ID NO: 15),
SSSSKAPPPS (SEQ ID NO: 16),
SRLPGPSDTPILPQ (SEQ ID NO: 17) and
GGGGSGGGGSGGGGSA (SEQ ID NO: 18).

In one embodiment, the GLP-1 receptor agonist is a full-length, truncated or variant of GLP-1 or GLP-1 analogue, for example, Exendin-4, GLP-1(1-36), GLP-1(1-37), GLP-1(7-36), GLP-1(7-37), liraglutide, lixisenatide, albiglutide, dulaglutide, or semaglutide.

In another embodiment, the fusion partner (FP) is a full-length, truncated or variants of immunoglobulin Fc fragment, albumin, XTEN or transferrin; preferably, the Fc fragment is derived from a human immunoglobulin Fc fragment; preferably, the Fc fragment is composed of one to four domains selected from the group consisting of CH1 domain, CH2 domain, CH3 domain and CH4 domain; preferably, the Fc fragment is derived from the Fc fragment of IgG, IgA, IgD, IgE, or IgM, more preferably IgG Fc fragment; more preferably, the Fc fragment is derived from the Fc fragment of IgG1, IgG2, IgG3, or IgG4, and more preferably, the IgG Fc fragment has reduced ADCC effect and/or CDC effect and/or enhanced binding affinity to FcRn.

In yet another embodiment, the fusion partner (FP) is also linked directly or via a second linker L2 to a ST sequence containing a sortase recognition site, and wherein the sortase is, for example, sortase A or sortase B; preferably, the ST contains the core recognition site LPXTG of sortase A, wherein X is any amino acid, and the ST is, such as LPETG, LPETGG or LPETGGG; more preferably, the ST further contains an affinity tag linked to the sortase recognition site, and the ST is, for example, LPETGGHHHHHH or LPETGGWSHPQFEK.

Wherein the second linker L2 is a peptide containing 1, 2, 3, 4, 5, 6, 7, 8, 9, 10, or more amino acids.

More preferably, the second linker L2 is a flexible peptide containing A, T, G, and/or S, such as $(GS)_n$, where n is an integer from 1 to 50, for example, 1, 2, 3, 4, 5, 6, 7, 8, 9, or 10; and most preferably, the second linker L2 is selected from the group consisting of:

GSGGGSGGGGSGGGGS (SEQ ID NO: 9),
GGGGSGGGGSGGGGSA (SEQ ID NO: 18),
GGGGS (SEQ ID NO: 19),
GGGGSGGGGS (SEQ ID NO: 20), and
GGGGSGGGGSGGGGS (SEQ ID NO: 21).

In another embodiment, the fusion protein has a structure of GLP-1-L1-FP-L2-ST, wherein GLP-1 represents the GLP-1 receptor agonist, and the GLP-1 receptor agonist, L1, L2, FP and ST have the same definition as described above, and wherein either or both of L1 and L2 may not be present.

In a specific embodiment, the amino acid sequence of the fusion protein is set forth in SEQ ID NO: 1, 3, 5, 7, or 22; or the fusion protein is encoded by the nucleic acid sequence set forth in SEQ ID NO: 2, 4, 6, 8, or 23.

In the second aspect of the present invention, a conjugate is provided, wherein one, two or more hydrophilic polymers are attached to the end of the fusion protein of the first aspect, preferably, the hydrophilic polymer is attached to the sortase recognition site via a transpeptidation reaction, in particular, the fusion protein may be in a form of monomer or dimer.

In one embodiment, the one, two or more hydrophilic polymers are each independently selected from polysaccharide and polyalkylene glycol, such as polypropylene glycol and polyethylene glycol; the polyalkylene glycol may be end-capped, for example, capped with alkoxy group such as methoxy group, and/or the polyalkylene glycol is linear or branched, and for example, the polyalkylene group is branched, such as branched polyethylene glycol, especially branched polyethylene glycol capped with methoxy group; the molecular weight of the polyalkylene glycol may be $\geq 1$, $\geq 10$, $\geq 20$, $\geq 30$, $\geq 40$, $\geq 50$, $\geq 60$, $\geq 70$, $\geq 80$, $\geq 90$, $\geq 100$, $\geq 110$, $\geq 120$, $\geq 130$, $\geq 140$, $\geq 150$, or $\geq 160$ kDa, for example, 5, 10, 20, 30, 40, 50, 60, 70, 80, 90, or 100 kDa, or a value between any two of the values; and preferably the molecular weight of the hydrophilic polymer is 20 kDa or 40 kDa.

The third aspect of the present invention is to provide a method for producing the conjugate described in the second aspect, which comprises contacting the fusion protein described in the first aspect with sortase (such as sortase A or sortase B) and the hydrophilic polymer, wherein one end of the hydrophilic polymer has an amino group that can be amidated by sortase.

In one embodiment, one end of the hydrophilic polymer contains poly-Gly, such as GGGAA.

The fourth aspect of the present invention is to provide a pharmaceutical composition comprising an effective amount of the fusion protein described in the first aspect and/or the conjugate described in the second aspect, and optionally a pharmaceutically acceptable carrier.

In one embodiment, the pharmaceutical composition is for use in reducing blood glucose or body weight, for example, in the treatment of diabetes, more specifically in the treatment of type I diabetes and/or type II diabetes, especially in the treatment of type II diabetes.

In another embodiment, the pharmaceutical composition is in a form of liquid preparation or lyophilized preparation.

The fifth aspect of the present invention is to provide uses of the fusion protein described in the first aspect or the conjugate described in the second aspect for the manufacture of a medicament for reducing blood glucose or body weight, for example, the medicament is for use in treatment of diabetes, including type I diabetes and type II diabetes, especially type II diabetes.

The sixth aspect of the present invention is to provide a method for reducing blood glucose or body weight, comprising administering the fusion protein described in the first aspect or the conjugate described in the second aspect to a subject in need thereof, for example, for treating diabetes, including type I diabetes and type II diabetes, especially type II diabetes.

The seventh aspect of the present invention is to provide a kit comprising the fusion protein described in the first aspect, the conjugate described in the second aspect, and/or the pharmaceutical composition described in the fourth aspect, and optional instructions for use.

The inventors found that the GLP-1-Fc conjugate of the present invention can provide a surprisingly long-term hypoglycemic effect. In addition, compared with the existing similar drugs, the GLP-1-Fc conjugate of the present invention can significantly reduce the rapid body weight loss caused by taking hypoglycemic drugs, so that the clinical safety of the drugs is greatly improved, and therefore has great value of clinical application.

## BRIEF DESCRIPTION OF DRAWINGS

FIG. 1A shows the structure of GLP-1 fusion protein eukaryotic expression vector and FIG. 1B shows the test results of growth curve and cell viability of GLP1-L1-Fc2-L2-ST stable cell line (clone number: P1-3G3C2) during fermentation.

FIG. 2 shows the microscopic examination results of GLP1-L1-Fc2-L2-ST stable cell line (clone number: P1-3G3C2) at different time points during fermentation. FIG. 2A is the image of cells on day 5 of cultivation, and FIG. 2B shows the cells when collected (day 13).

FIG. 3 shows the glucose metabolism in the culture supernatant of GLP1-L1-Fc2-L2-ST stable cell line (clone number: P1-3G3C2) during fermentation.

FIG. 4 shows the metabolisms of $NH_4^+$ and lactose (Lac) in the culture supernatant of GLP1-L1-Fc2-L2-ST stable cell line (clone number: P1-3G3C2) during fermentation.

FIG. 5 shows the electrophoresis results of GLP1-L1-Fc2-L2-ST protein in non-reduced gel (left: M/1/2/3) and reduced gel (right: 4/5/6/M). 1 and 6: supernatant of terminal fermentation broth; 3 and 4: samples after purification; 2 and 5: fermentation broth samples on day 10.

FIG. 6 shows the test results of SDS electrophoresis (A) and SEC analysis (B) of purified GLP1-L1-Fc2-L2-ST protein.

FIG. 7 shows the test results of SDS electrophoresis and RPC analysis of the crosslinking rate of two proteins. FIG. 7A and FIG. 7C are the SDS gel of GLP1-L1-Fc2-L2-ST protein crosslinked with 20kDa and 40kDa PEG, respectively; FIG. 7B and FIG. 7D are the RPC results of GLP1-L1-Fc2-L2-ST protein crosslinked with 20kDa and 40kDa PEG, respectively; and FIG. 7E is the RPC result of GLP1-L1-Fc2-ST protein crosslinked with 40kDa PEG.

FIG. 8 shows the results of the anion exchange chromatogram (A) and RPC test (B) of GLP1-L1-Fc2-L2-ST after crosslinking.

FIG. 9 shows the activity test results of GLP1-L1-Fc2-L2-ST crosslinked with 20kDa PEG and 40kDa PEG, respectively. Compared with dulaglutide, the relative activity of non-crosslinked substrate (GLP1-Fc) is 98.10%. The activities of both PEG-crosslinked products decreased, wherein the relative activity of the product crosslinked with 20kDa PEG (PEG (20kDa)-GLP1-Fc) is 40.38% and the relative activity of the product crosslinked with 40kDa PEG (PEG (40kDa)-GLP1-Fc) is 32.85%.

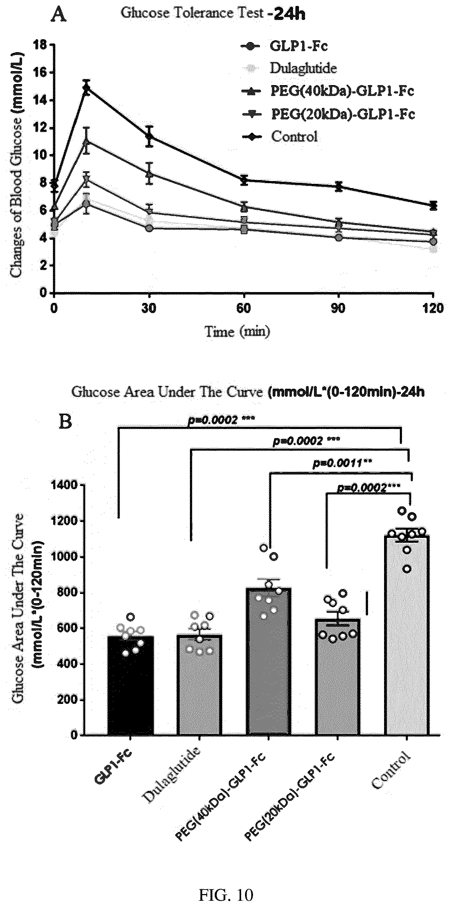

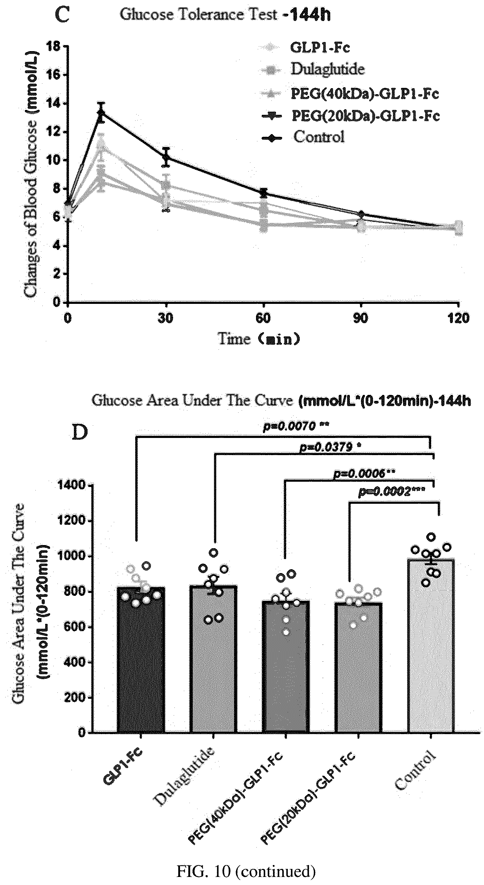

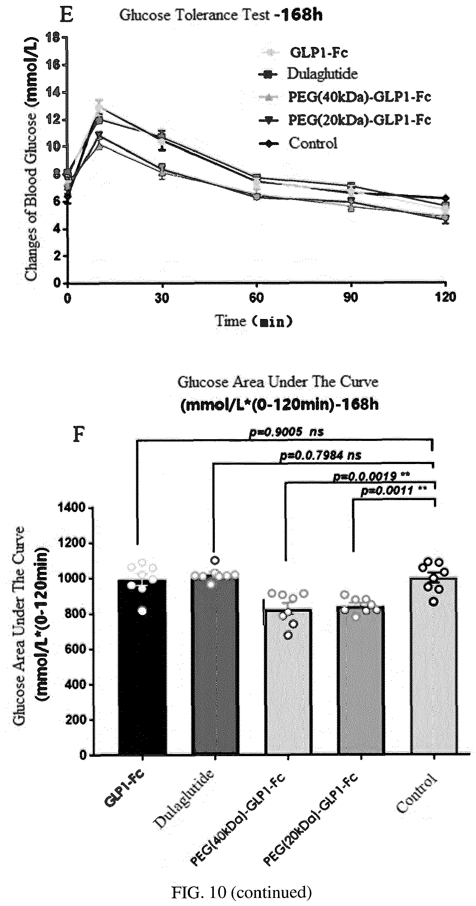

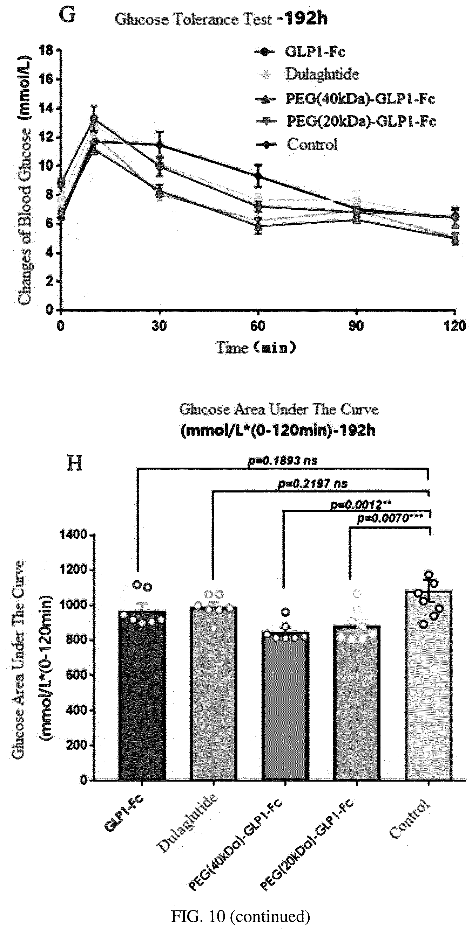

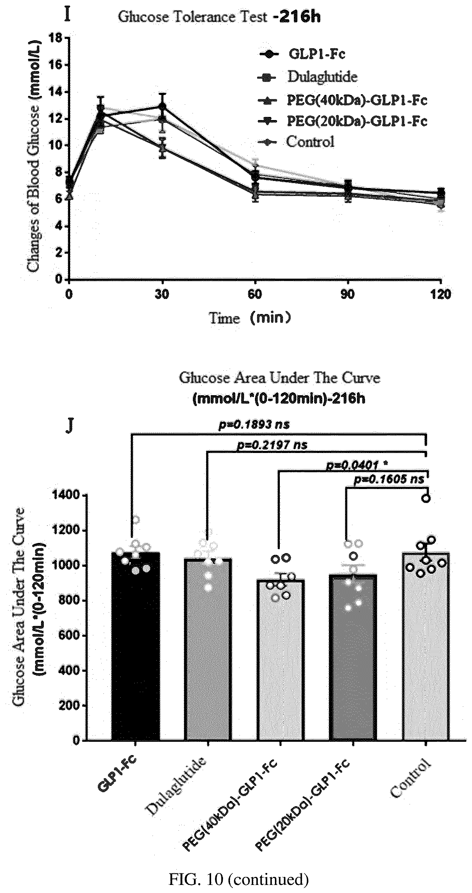

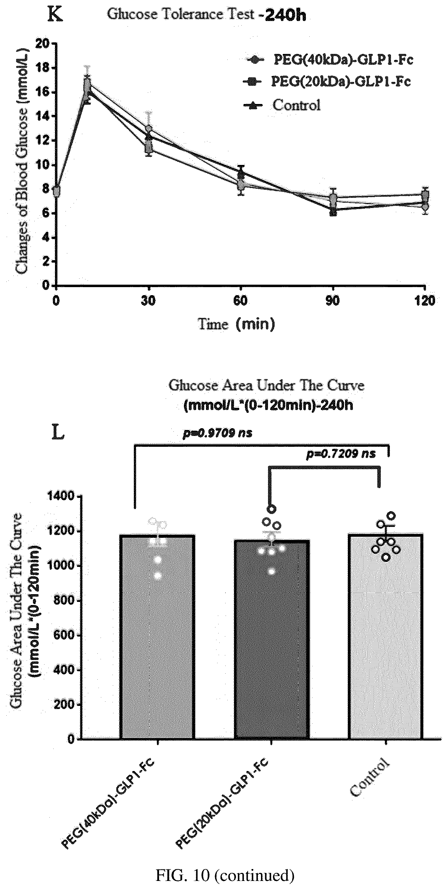

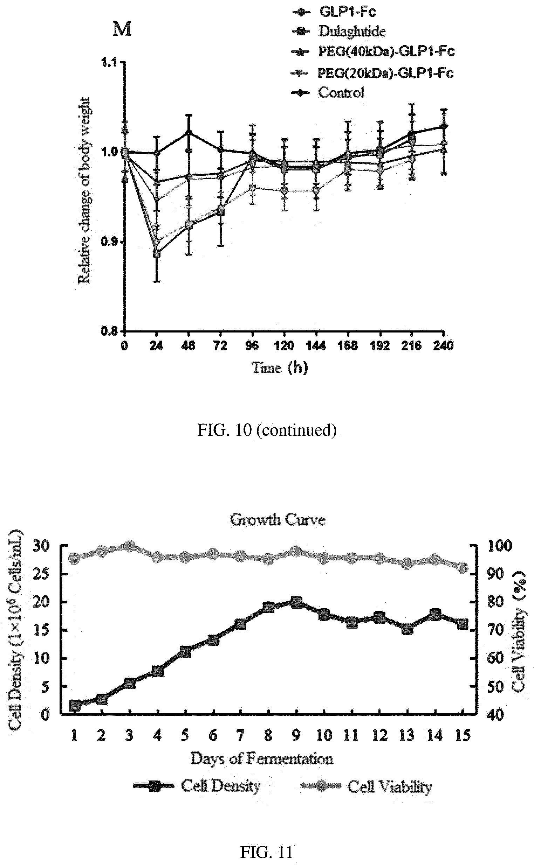

FIG. 10A shows the response curve of glucose tolerance test in C57BL/6 mice after 24h of administration; FIG. 10B shows the effect of 24h after administration on the glucose tolerance of C57BL/6 mice (AUC); FIG. 10C shows the response curve of glucose tolerance test in C57BL/6 mice after 144h of administration; FIG. 10D shows the effect of 144h after administration on the glucose tolerance of C57BL/6 mice (AUC); FIG. 10E shows the response curve of glucose tolerance test in C57BL/6 mice after 168h of administration; FIG. 10F shows the effect of 168h after administration on the glucose tolerance of C57BL/6 mice (AUC); FIG. 10G shows the response curve of glucose tolerance test in C57BL/6 mice after 192h of administration; FIG. 10H shows the effect of 192h after administration on the glucose tolerance of C57BL/6 mice (AUC); FIG. 10I shows the response curve of glucose tolerance test in C57BL/6 mice after 216h of administration; FIG. 10J shows the effect of 216h after administration on the glucose tolerance of C57BL/6 mice (AUC); FIG. 10K shows the response curve of glucose tolerance test in C57BL/6 mice after 240h of administration; FIG. 10L shows the effect of 240h after administration on the glucose tolerance of C57BL/6 mice (AUC); and FIG. 10M shows the body weight changes of C57BL/6 mice after administration.

FIG. 11 shows the growth curve and cell viability of GLP1-L1-Fc4-L2-STG3 stable cell line (clone number: P1-P132C7) during fed-batch fermentation.

FIG. 12 shows the microscopic images of GLP1-L1-Fc4-L2-STG3 stable cells (clone number: P1-P132C7) at different time points during fed-batch fermentation. FIG. 12A shows the cells on day 5 of cultivation, and FIG. 12B shows the cells when collected (day 13).

FIG. 13 shows the glucose metabolism in the culture supernatant of GLP1-L1-Fc4-L2-STG3 stable cell line (clone number: P1-P132C7) during fed-batch fermentation.

FIG. 14 shows the pH trend of GLP1-L1-Fc4-L2-STG3 stable cell line (clone number: P1-P132C7) in a 15L bioreactor during fed-batch fermentation.

FIG. 15 shows the SDS-PAGE electrophoresis results of the target protein GLP1-L1-Fc4-L2-STG3 in the supernatant of the 15L fed-batch fermentation broth. Lane 1 is the purified sample, lane 2 is protein marker, and lanes 3, 4 and 5 are the supernatant of fermentation broth on the 12th, 13th and 14th day.

FIG. 16 shows the results of SDS-PAGE electrophoresis (A) and SEC analysis (B) of purified GLP1-L1-Fc4-L2-STG3 protein.

FIG. 17 shows the results of SDS-PAGE electrophoresis and RPC analysis of the crosslinking rate of two proteins. FIG. 17A is the SDS test of GLP1-L1-Fc4-L2-STG3 protein crosslinked with 40kDa PEG, and FIG. 17B is the RPC test of GLP1-L1-Fc4-L2-STG3 protein crosslinked with 40kDa PEG.

FIG. 18 shows the results of the anion exchange chromatogram (A) and SEC test (B, C) of GLP1-L1-Fc4-L2-STG3 after crosslinking.

FIG. 19 shows the activity test results of GLP1-L1-Fc4-L2-STG3 crosslinked with 40kDa PEG. The relative activity of non-crosslinked substrate (GLP1-Fc) to dulaglutide is 112.24%. The activity of PEG-crosslinked product decreased, wherein the relative activity of GLP1-L1-Fc4-L2-STG3 crosslinked with 40kDaPEG (PEG (40kDa)-GLP1-Fc) is 21.51%.

FIG. 20A shows the glucose tolerance of C57BL/6 mice (AUC) 1-8 days after administration, and FIG. 20B shows the body weight changes of C57BL/6 mice after administration.

FIG. 21A shows the effect on blood glucose of STZ-induced type II diabetic mice1-12 days after administration, and FIG. 21B shows the body weight changes of type II diabetic mice after administration.

## DETAILED DESCRIPTION

The term used in this application has the same meaning as in the prior art. In order to define the meaning of the terms used, the specific meanings of some terms in this application are given below. When the definition herein conflicts with the conventional meaning of the term, the definition herein shall prevail.

The term "amino acid" as used herein refers to natural amino acids, unnatural amino acids, and amino acid analogs and all their D- and L-stereoisomers. Unnatural amino acid includes, but is not limited to, azetidine carboxylic acid, 2-aminoadipate, 3-aminoadipate, β-alanine, aminopropionic acid, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, tert-butylglycine, 2,4-diaminoisobutyric acid, 2,2'-diaminopimelic acid, 2,3-diaminopropionic acid, N-ethyl glycine, N-ethyl asparagine, homoproline, hydroxylysine, allo-hydroxylysine, 3-hydroxyproline, 4-hydroxyproline, isodesmosine, allo-isoleucine, N-methylalanine, N-methylglycine, N-methylisoleucine, N-methylpentylglycine, N-methylvaline, naphthalanine, norvaline, norleucine, ornithine, pentylglycine, pipecolic acid and thioproline. Amino acid analogs include natural amino acids and unnatural amino acids whose C-terminal carboxyl group, N-terminal amino group or side chain group is chemically end-capped reversibly or irreversibly, or chemically modified into another functional group, such as methionine sulfoxide, methionine sulfone, S-(carboxymethyl)-cysteine, S-(carboxymethyl)-cysteine sulfoxide and S-(carboxymethyl)-cysteine sulfone.

The term "polypeptide" or "protein" used herein interchangeably refers to a compound of at least two amino acid residues connected to each other by covalent bonds (such as peptide bonds), which may be recombinant polypeptides, natural polypeptides or synthetic polypeptides. In addition, the "polypeptide" or "protein" of the present invention also refers to variants or analogs thereof, that is, polypeptides varying in the amino acid sequence by one or more substitutions, deletions, insertions, fusions, truncations, or any combination thereof. A polypeptide variant may be fully functional or may lack one or more activities. Fully functional variants may contain, for example, only conservative substitutions or changes at non-critical residues or in non-critical regions. Functional variants may also contain substitutions of similar amino acids, which result in unchanged or insignificant changes in function. Amino acids essential for function can be identified by methods known in the art, such as site-directed mutagenesis or glycine scanning mutagenesis (Cunningham, B. and Wells, J., Science, 244: 1081-1085, 1989). The key sites for polypeptide activity can be determined, for example, by structural analysis such as crystallization, nuclear magnetic resonance or photoaffinity label (Smith, L., et al., J. Mol. Biol., 224:899-904, 1992; and de Vos, A. et al., Science, 255: 306-312, 1992).

The term "conjugate" used herein refers to a product formed by a polypeptide or a polypeptide variant covalently or non-covalently linked to the modification group described herein.

Generally, the term "polymer" used herein has the meaning commonly understood by those of ordinary skill in the art, and refers to both the polymer itself and its terminal-modified derivatives, unless explicitly stated otherwise.

In addition, for polymer such as polyethylene glycol, there are many ways to determine their molecular weight. Since polymers are composed of molecules with different degrees of polymerization within a certain distribution range, the molecular weights of the polymers are generally represented by their average molecular weight, specifically, number-average molecular weight or weight-average molecular weight. Although the number-average molecular weight and weight-average molecular weight may have some deviations when the degree of polymerization of the polymers differs greatly, for polymers with a narrow distribution range, the two tend to be equal. Unless otherwise specified, for polymers such as polyethylene glycol mentioned herein, when referring to its molecular weight, it can be either weight-average molecular weight or number-average molecular weight.

GLP-1 Receptor Agonist

Glucagon-like peptides include glucagon-like peptide-1 (GLP-1) and glucagon-like peptide-2 (GLP-2), both of which are derived from proglucagon, which consists of 158 amino acids and may be cleaved into different peptide chains. Since GLP-1 has the pharmacological effects of promoting insulin secretion, protecting islet β-cells, inhibiting glucagon secretion and gastric emptying, and reducing appetite, it can be clinically used in the treatment of type II diabetes and obesity. GLP-2 is a nutritional factor that promotes the growth of the small intestine, inhibits cell apoptosis, promotes gastric emptying, and increases appetite, so it can be clinically used in the treatment of short-bowel syndrome. The biologically active GLP-1 in the human body is mainly GLP-1(7-36) amide and GLP-1(7-37). Natural GLP-1 (with half-life of less than 5min) is easily hydrolyzed and inactivated by dipeptidyl peptidase IV (DPP-IV), so it is difficult to be used in clinic.

The term "GLP-1 receptor agonist" refers to a molecule capable of activating the GLP-1 receptor, such as naturally occurring GLP-1 derived from different sources such as human or animals, and the functional variants, fragments and analogs formed by one or more (such as 1, 2, 3, 4, 5, 6, 7, 8, 9, 10, 11, 12, 13, 14, 15, 16, 17, 18, 19, 20 or more) amino acid substitutions, deletions and/or additions of the wild-type sequences of natural GLP-1 receptor agonists (such as GLP-1 and exendin-4), for example, liraglutide, lixisenatide, albiglutide, dulaglutide, semaglutide, etc.

GLP-1 Fusion Protein

GLP-1 receptor agonist (such as GLP-1 and functional variants and fragments thereof) is linked to a fusion partner capable of increasing the half-life, such as the Fc fragment of human immunoglobulin, to form a fusion protein. The fusion proteins in the present invention are refer to as "fusion protein with GLP-1 receptor agonist activity" or "GLP-1 fusion protein".

Human immunoglobulin IgG is composed of four polypeptides (two identical copies of light chain and heavy chain) covalently linked by disulfide bonds. The proteolysis of IgG molecules by papain produces two Fab fragments and one Fc fragment. The Fc fragment is composed of two polypeptides linked together by disulfide bonds. Each polypeptide, from N- to C-terminal, consists of hinge region, CH2 domain and CH3 domain. The structure of the Fc fragment is almost the same in all subtypes of human immunoglobulin. IgG is one of the most abundant proteins in human blood, which constitutes 70% to 75% of total immunoglobulin in human serum.

The circulating half-life of IgG, which is the longest among all five types of immunoglobulins, can reach 21 days. It has been reported to combine the Fc region of IgG with other proteins (such as various cytokines and soluble receptors) to form fusion proteins (Capon et al., Nature, 337:525-531, 1989; Chamow et al., Trends Biotechnol., 14: 52-60, 1996; and U.S. Patent Nos. 5,116,964 and 5,541,087). A typical fusion protein is a dimeric protein linked by the cysteine residues in the IgG Fc hinge region to form a molecule similar to IgG but lacking CH1 domain and light chain. Due to the structural homology, the *in vitro* pharmacokinetic properties of the Fc fusion protein are quite similar to those of the isotype of human IgG. Therefore, constructing hGH fusion proteins linked to the Fc region of human IgG will help extend the circulating half-life of hGH and/or increase its biological activity.

The Fc region of immunoglobulin is safe to be used as a pharmaceutical carrier because it is a biodegradable polypeptide that can be metabolized in the body. In addition, compared with the entire immunoglobulin molecule, the Fc region of immunoglobulin has a relatively low molecular weight, which is beneficial to the preparation, purification and production of the conjugate. Since the immunoglobulin Fc region does not contain Fab fragment (its amino acid sequence varies according to the antibody subclass and is therefore highly heterogeneous), it is expected that the immunoglobulin Fc region can greatly increase the homogeneity of the substance and have low antigenicity.

The term "Fc region of immunoglobulin" or "Fc fragment" as used in the present invention refers to a protein containing heavy chain constant region 2 (CH2) and heavy chain constant region 3 (CH3), but not variable region of heavy chain and light chain, heavy chain constant region 1 (CH1) and light chain constant region 1 (CL1). It may also contain the hinge region in the heavy chain constant region. Furthermore, the Fc fragment used in the present invention may contain part or all of the Fc region containing heavy chain constant region 1 (CH1) and/or the light chain constant region 1 (CL1) without variable regions of heavy chain and light chain, as long as it has a physiological function that is basically similar to or better than that of natural protein. Moreover, it may be a Fc fragment with a relatively long deletion the amino acid sequences of CH2 and/or CH3. Hence, the immunoglobulin Fc region used in the present invention may comprise 1) CH1 domain, CH2 domain, CH3 domain and CH4 domain; 2) CH1 domain and CH2 domain; 3) CH1 domain and CH3 domain; 4) CH2 domain and CH3 domain; 5) the combination of one or more domains with (part or all of) the immunoglobulin hinge region; or 6) the dimer of any domains of heavy chain constant region and light chain constant region.

Besides, the immunoglobulin Fc region of the present invention comprises natural amino acid sequence and derivatives (mutants) thereof. Owing to one or more deletions, insertions, non-conservative or conservative substitutions, or combination thereof of amino acid residues, the amino acid sequence derivative may have a sequence different from the natural amino acid sequence. For example, for IgG Fc, amino acid residues at positions 214 to 238, 297 to 299, 318 to 322, or 327 to 331 that are known critical to binding can be used as suitable targets for modification. The immunoglobulin Fc region of the present invention may also comprise a variety of other derivatives, including those without the region capable of forming disulfide bonds, those having several amino acid residues deletion at the N-terminal of the natural Fc, or those having additional methionine residues to the N-terminal of the natural Fc. In addition, to get rid of effector functions, deletion may be designed at complement binding site, such as C1q binding site and ADCC site. Techniques for preparing such derivatives of the immunoglobulin Fc region are disclosed in WO 97/34631 and WO 96/32478.

For proteins and peptides, amino acid substitutions that generally do not change the molecular activity are known in the art (H.Neurath, RL Hill, The Proteins, Academic Press, New York, 1979). The most common substitutions are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu and Asp/Gly, in either way.

If necessary, the Fc region is allowed to be modified, such as phosphorylation, sulfation, acrylate, glycosylation, methylation, farnesylation, acetylation, and amidation.

The Fc derivatives have the same biological activity as the Fc region in the present invention or have improved structural stability (such as structural stability to heat, pH, etc.) than the corresponding Fc region thereof.

In addition, these Fc regions can be derived from natural forms isolated from human and other animals including cattle, goat, pig, mouse, rabbit, hamster, rat and guinea pig, or derived from recombinant or derivative of transformed animal cells or microorganisms. Herein, the Fc region can be obtained from natural immunoglobulin by separating intact immunoglobulin from human or animal organisms and treating them with proteolytic enzymes. Papain digests natural immunoglobulin into Fab and Fc regions, while pepsin treatment results in the production of pFc' and F(ab')$_2$ fragments. Fc or pFc' fragments can be isolated, e.g., by size exclusion chromatography.

In addition, the immunoglobulin Fc region of the present invention may be a form having natural sugar chains, or increased or reduced sugar chains compared to the natural form, or may be a deglycosylated form. The increase, decrease or removal of immunoglobulin Fc sugar chains can be accomplished by methods commonly used in the art, such as chemical methods, enzymatic methods, and genetic engineering methods using microorganisms. Removal of sugar chains from the Fc fragment results in a significant reduction in binding affinity to complement (C1q) and reduction or loss of antibody-dependent cell-mediated cytotoxicity or complement-dependent cytotoxicity, and thereby unnecessary *in vivo* immune responses will not be induced. In view of this, the immunoglobulin Fc region in deglycosylated or unglycosylated form may be more suitable for the purpose of the present invention for use as a medicament.

The term "deglycosylation" as used in the present invention means the enzymatic removal of sugar moieties from the Fc region, and the term "unglycosylated" means that the Fc region is produced in an aglycosylated form (for example, in eukaryotic cells such as mammalian cells or in prokaryotes such as *E. coli).*

In addition, the immunoglobulin Fc region may be an Fc region derived from IgG, IgA, IgD, IgE, and IgM, or prepared by a combination or hybrid thereof. Preferably, it is derived from IgG or IgM (two of the most abundant proteins in human blood), most preferably IgG (which is known to extend the half-life of ligand-binding protein).

The term "combination" as used in the present invention means a dimer or a multimer formed by two or more single-chain polypeptides which are linked together, where the single-chain polypeptides can be derived from the same or different immunoglobulin Fc region. That is, the dimer or the multimer may be formed by two or more fragments selected from the group consisting of IgG Fc fragment, IgA Fc fragment, IgM Fc fragment, IgD Fc fragment, and IgE Fc fragment.

In another embodiment, the GLP-1 fusion protein of the present invention is a homodimer.

In another embodiment, the GLP-1 fusion protein is further linked to a fragment containing a sortase recognition site, i.e., the "ST" fragment described in the present invention.

The term "sortase", including sortase A and sortase B, was first discovered to have the function of anchoring bacterial surface proteins on the cell wall. The "sorting signal" of the surface proteins was identified to consist of three key parts: the LPXTG sequence, the hydrophobic sequence and a tail of positively charged residues in most cases. The LPXTG sequence is very conservative and can be recognized by sortase, then the cysteine (Cys) of the transpeptidase acts as a nucleophilic group to attack the peptide bond between the threonine and glycine of the C-terminal motif LPXTG of the substrate (such as surface protein), causing the peptide bond to cleave (acylation), which in turn produces an acylase intermediate, and finally the peptide is covalently linked to the cell wall or fimbriae subunit to function (deacylation).

In one embodiment of the present invention, sortase A is utilized for site-directed coupling of PEG. In a specific embodiment, the sortase A core recognition sites LPETG, LPETGG, and LPETGGG are used. In another specific embodiment, different affinity tags can be added to the recognition site, such as LPETGGHHHHHH, LPETGGWSHPQFEK, etc.

In another embodiment, the GLP-1 fusion protein of the present invention is produced by recombinant methods, comprising, for example, expressing the protein in a suitable prokaryotic or eukaryotic host cell, and isolating the GLP-1 fusion protein of the present invention by conventional techniques. For example, the nucleotide sequence encoding the peptide can be synthesized by chemical synthesis method first, and then the sequence can be cloned into a suitable expression vector for expression under the control of a suitable promoter. Alternatively, mutagenesis methods such as PCR mutagenesis can be employed to obtain the nucleotide sequence encoding GLP-1 from wild-type GLP-1, and then the sequence and the sequence of other elements for constructing the fusion protein can be cloned to a suitable expression vector for expression under the control of a suitable promoter. These technologies are completely within the abilities of those of ordinary skill in the art, and there are numerous teachings in the prior art.

Suitable eukaryotic host cells comprise mammalian cells, such as CHO, COS, HEK 293, BHK, SK-Hep and HepG2. The cells are preferably growing under conditions suitable for expressing the GLP-1 fusion protein of the present invention. As for the reagents and conditions used to produce or isolate the GLP-1 fusion protein of the present invention, there are no special restrictions, and any system known in the art or commercially available can be applied. In a preferred embodiment, the GLP-1 fusion protein is obtained by methods described in the art.

There are a variety of expression vectors that can be used to prepare the GLP-1 fusion protein, which can be selected from eukaryotic and prokaryotic expression vectors. Prokaryotic expression vectors may include, for example, plasmids such as pRSET, pET, and pBAD, in which promoters that can be used include, for example, lac, trc, trp, recA, or araBAD. Eukaryotic expression vectors include: (i) vectors used for expression in yeast such as pAO, pPIC, pYES, and pMET, in which promoters such as AOX1, GAP, GAL1, AUG1, etc. can be used; (ii) vectors used for expression in insect cells such as pMT, pAc[delta], pIB, pMIB, pBAC, etc., in which promoters such as PH, p10, MT, Ac5, OplE2, gp64, polh, etc. can be used; and (iii) vectors used for expression in mammalian cells such as pSVL, pCMV, pRc/RSV, pcDNA3, pBPV, etc., and vectors derived from viral systems such as vaccinia virus, adeno-associated virus, herpes virus, retrovirus, and the like, in which promoters such as CMV, SV40, EF-1, UbC, RSV, ADV, BPV and $\beta$-actin can be used. In a preferred embodiment, the GLP-1 fusion protein is expressed in a prokaryotic or eukaryotic cell system, and codon-optimized encoding sequences are used. In a preferred embodiment, the sequence for expressing the GLP-1 fusion protein contains a leader peptide and/or a signal peptide to facilitate the secretion of the GLP-1 fusion protein from the cell to the outside of the cell for separation and purification. In another preferred embodiment, the sequence expressing the GLP-1 fusion protein does not contain a leader peptide and/or a signal peptide, and instead of being secreted outside the cell, the GLP-1 fusion protein is obtained by lysing the cell for separation and purification.

In another embodiment, in the fusion protein of the present invention, the GLP-1 protein and the Fc fragment are directly linked. In another embodiment, the GLP-1 protein and the Fc fragment are linked through a first linker L1. In another embodiment, the Fc fragment and the fragment containing the sortase recognition sequence are directly linked. In another embodiment, the Fc fragment and the fragment containing the sortase recognition sequence are linked through a second linker L2.

In a preferred embodiment, the first linker L1 and/or the second linker L2 is a peptide containing 1, 2, 3, or more amino acids. More preferably, the first linker L1 and/or the second linker L2 is a flexible peptide containing A, T, G and/or S, such as $(GS)_n$, where n is an integer from 1 to 50, for example 1, 2, 3, 4, 5, 6, 7, 8, 9, or 10.

For example, the first linker L1 is selected from the group consisting of:

GSGGGSGGGGSGGGGS (SEQ ID NO: 9),
GSGGGGSGGGGSGGGGSGGGGSGGGGS (SEQ ID NO: 10),
GGGGSGGGGSGGGGSGGGGS (SEQ ID NO: 11),
GSGGGGSGGGGSGGGGSGGGGSGGGGSGGGGSGGGGS (SEQ ID NO: 12),
GGGSGGGSGGGSGGGSGGGS (SEQ ID NO: 13),
PRFQDSSSSKAPPPSLPSPSRLPGPSDTPILPQ (SEQ ID NO: 14),
SSSSKAPPPSLPSPSRLPGPSDTPILPQ (SEQ ID NO: 15),
SSSSKAPPPS (SEQ ID NO: 16),
SRLPGPSDTPILPQ (SEQ ID NO: 17) and
GGGGSGGGGSGGGGSA (SEQ ID NO: 18).

For example, the second linker L2 is selected from the group consisting of:

GSGGGSGGGGSGGGGS (SEQ ID NO: 9),
GGGGSGGGGSGGGGSA (SEQ ID NO: 18),
GGGGS (SEQ ID NO: 19),
GGGGSGGGGS (SEQ ID NO: 20) and
GGGGSGGGGSGGGGS (SEQ ID NO: 21).

GLP-1 Fusion Protein Conjugates

The GLP-1 fusion protein of the present invention can be conjugated with one or more polymer groups to form a GLP-1 fusion protein conjugate, wherein the polymer is conjugated to the end of the fusion protein, such as the N-terminal or the C-terminal. The "polymer" used herein is preferably physiologically acceptable, which includes those soluble in an aqueous solution or suspension and without negative effects such as side effects on mammals after administration of the GLP-1 fusion protein conjugate in a pharmaceutically effective amount. The polymers that can be used in the present invention are not particularly limited. The polymers generally preferably have 2 to about 3000 repeating units. The polymer group can be selected from natural or synthetic polymers, examples of which include, but are not limited to, for example, polysaccharides, polyalkylene glycols such as polyethylene glycol (PEG), polypropylene glycol (PPG), polyethylene oxide (PEO), copolymer of ethylene glycol and propylene glycol, polyvinyl alcohol and any combination thereof. In a preferred embodiment, the GLP-1 fusion protein conjugate of the present invention is conjugated with one or more PEG groups for modification.

In the present invention, the polymer is not limited to a particular structure, it can be linear (such as alkoxy PEG or bifunctional PEG), branched or multi-armed (such as bifurcated PEG or PEG linked to polyol core), dendritic or may have degradable linkages. In addition, the internal structure of the polymer can be organized in any number of different patterns, which can be selected from homopolymers, alternating copolymers, random copolymers, block copolymers, alternating terpolymers, random terpolymers, block terpolymers, and the like. The polymer may also include poly(alkylene oxide) polymers, polymaleic acid, poly(D,L-alanine), and the like.

In some embodiments, the polymer is polyethylene glycol (PEG) or derivatives thereof such as methoxy polyethylene glycol (mPEG). Herein, unless specifically indicated, the polyethylene glycols (PEG) include those either with hydroxyl groups as the terminal group or with other groups as the terminal group. The other groups include, but are not limited to, alkoxy, cycloalkoxy, cycloalkyloxy, alkenyl, aryloxy or aralkyloxy. These PEG molecular types are known in the prior art and are routinely used in polypeptide modification. The PEG side chain can be linear, branched, bifurcated or composed of multiple arms. Different polyethylene glycols can have different polymer chain lengths and polymer structures.

In the present invention, there is no particular limitation on the molecular weight of PEG, and its molecular weight can range from 0.1 to 200 kDa, such as 1 to 150 kDa, 2 to 100 kDa, 3 to 80 kDa, 4 to 50 kDa, or 5 to 40 kDa. Other useful PEG include, for example, those disclosed in WO 03/040211, US 6,566,506, US 6,864,350, and US 6,455,639. In particular, the PEG has the general formula $HO\text{-}CH_2CH_2O\text{-}(CH_2CH_2O)_n\text{-}CH_2CH_2\text{-}OH$, wherein the range of n is about 5 to 4000. As mentioned above, the PEG of the present invention includes PEG with other terminal groups, such as methoxy PEG, branched PEG, bifurcated PEG, and the like. Suitable branched PEGs can be prepared as described in US Patent No. 5,932,462. The bifurcated PEG refers to a PEG that has a branch near one end of the polymer chain, and the main chain of the bifurcated PEG can be linear or branched.

It is known to those skilled in the art that in a bioactive molecule conjugated with a polymer group, as the molecular weight of the polymer group increases, the biological activity of the conjugate gradually decreases (Bailon et al. al. Rational design of potent, long-lasting form of interferon: A 40 kDa branched polyethylene glycol-conjugated interferon $\alpha$-2a for the treatment of hepatitis C. Bioconjugate Chem 2001; 12: 195-202; Bowen et al. Relationship between molecular mass and duration of activity of polyethylene glycol conjugated granulocyte colony-stimulating factor mutein. Experimental Hematology 1999; 27: 425-32; and Bailon et al. PEG-modified biopharmaceuticals. Expert Opin Deliv. 2009; 6: 1-16). It is also known to those skilled in the art that as the molecular weight of the polymer group increases, the biological half-life and/or plasma half-life of the conjugate increases accordingly.

In order to provide a stable therapeutic effect over a long period, and to reduce the frequency of administration to improve patient compliance, it is desirable to extend the biological half-life of GLP-1 fusion protein conjugate as much as possible while retaining significant activity of GLP-1 receptor agonist. Therefore, in one embodiment, the present invention provides GLP-1 fusion protein conjugates with extended biological half-life and significant activity of GLP-1 receptor agonist.

In a specific embodiment, in the GLP-1 fusion protein conjugate of the present invention, the molecular weight of the one or more polymer groups (such as PEG) is $\geq 1$, $\geq 10$, $\geq 20$, $\geq 30$, $\geq 40$, $\geq 50$, $\geq 60$, $\geq 70$, $\geq 80$, $\geq 90$, $\geq 100$, $\geq 110$, $\geq 120$, $\geq 130$, $\geq 140$, $\geq 150$, or $\geq 160$ kDa, for example, 5, 10, 20, 30, 40, 50, 60, 70, 80, 90, or 100 kDa, or a value between any two of the above values. It should be noted that when describing the molecular weight of the conjugated polymer group in a GLP-1 fusion protein conjugate, if there are multiple conjugated polymer groups in the conjugate, the sum of the molecular weights of all conjugated polymer groups in the conjugate is calculated, unless otherwise specified.

The polymer used in the present invention is known in the prior art, and it can be obtained through a variety of ways, including, for example, commercial routes, such as CarboMer, Inc., JT Baker, The Dow Chemical Company, etc., or it can be prepared by oneself according to methods known in the art, for example as described in EP1245608. The present invention is not limited to polymers made by any specific method.

After the conjugation reaction, the conjugate can be separated by a suitable method, including, for example, ultrafiltration, dialysis, or chromatography, etc., all of which are within the abilities of those of ordinary skill in the art.

Pharmaceutical Composition

The GLP-1 fusion protein or GLP-1 fusion protein conjugate of the present invention can have multiple applications, including, for example, for use in reducing blood glucose. Therefore, the present invention also provides a pharmaceutical composition for reducing blood glucose, which comprises a therapeutically effective amount of the fusion protein or conjugate of the present invention, and optionally a pharmaceutically acceptable carrier. Preferably, the pharmaceutical composition can be used for the treatment of diabetes, more preferably for the treatment of type I diabetes and/or type II diabetes, and particularly preferably for the treatment of type II diabetes.

The therapeutically effective amount of the fusion protein or conjugate of the present invention depends on the route of administration, the type of subjects, and the physical characteristics of the specific mammal considered. These factors and their relationship with determining the amount are well known to those skilled in the medicinal field. The amount and method of administration can be adjusted to achieve optimal efficacy to deliver the peptide to the subject, but depending on factors well known to those skilled in the medicinal field such as body weight, diet, concomitant medication, and other factors.

The pharmaceutical composition of the present invention can be administered in combination therapy, that is, in combination with one or more other agents, wherein the pharmaceutical composition and the other agents are administered together or sequentially. In other embodiments, the other agents may be administered before, during, or after the administration of one or more of the fusion protein, conjugate, or pharmaceutical composition thereof of the present invention. The other agents that can be used in the present invention include, for example, agents capable of reducing blood glucose, such as insulin, insulin analogs, dextrin agonists, and cholecystokinin, and/or other compounds or compositions for treating diseases. Preferably, such a combination administration can achieve combined or even synergistic effects.

As used herein, "pharmaceutically acceptable carrier" or "physiologically acceptable carrier" can be used interchangeably, including one or more of any and all physiologically compatible salts, solvents, dispersion media, coatings, antibacterial and antifungal agents, isotonic and absorption delaying agents, etc. In some embodiments, the carrier is suitable for intravenous, intramuscular, subcutaneous, parenteral, spinal or epidermal administration (e.g., by injection or infusion). Depending on the route of administration, the therapeutic agent may be coated with certain materials to protect the therapeutic agent from the action of acids and other natural conditions that may inactivate the therapeutic agent.

When administered, the pharmaceutical preparation of the present invention is administered in a pharmaceutically acceptable amount in a pharmaceutically acceptable composition. The term "pharmaceutically acceptable" means a non-toxic substance that does not interfere with the biologically active efficacy of the active components. Such preparations generally contain salts, buffers, preservatives, compatible carriers, and optionally other therapeutic agents, such as supplementary immune enhancers, including adjuvants, chemokines and cytokines. When used in medicaments, the salt should be pharmaceutically acceptable. However non-pharmaceutically acceptable salts can be conveniently used to prepare pharmaceutically acceptable salts thereof, thus they are not excluded from the scope of the present invention.

If necessary, the GLP-1 fusion protein or GLP-1 fusion protein conjugate of the present invention can be combined with pharmaceutically acceptable carriers. The term "pharmaceutically acceptable carriers" used herein refers to one or more compatible solid or liquid fillers, diluents, or encapsulating substances, which are suitable for administration to mammals such as human. The term "carrier" means organic or inorganic, natural or synthetic components, which are combined with the active components to facilitate application. The components of the pharmaceutical composition can also be blended in a form in which there are no interactions that can significantly disrupt the therapeutic effect of the desired drug.

Preferably, the pharmaceutical composition of the present invention may contain a buffer system, and preferably the buffer system is an acetate buffer solution with a pH of about 3.0 to about 6.0, or a phosphate buffer solution with a pH of about 5.0 to about 9.0. In some specific embodiments, suitable buffers include acetate, citrate, borate, and phosphate.

Optionally, the pharmaceutical composition may also contain suitable preservatives, such as benzalkonium chloride, chloro-tert-butanol, parabens and thimerosal.

The pharmaceutical composition can be conveniently presented in a unit dosage form and can be prepared by any known method in the pharmaceutical field. The method comprises the step of combining the active agent with a carrier, which contains one or more accessory ingredients. Generally, the composition is prepared by intimately combining the active compound with one or both of a liquid carrier and a finely divided solid carrier, followed by shaping the product if necessary.

Pharmaceutical compositions suitable for parenteral administration may be sterile aqueous or non-aqueous formulations containing one or more fusion proteins or conjugates. In some embodiments, the formulation is isotonic with the blood of the subject. This preparation can be formulated according to known methods using suitable dispersing or wetting agents and suspending agents. The sterile injection preparation can also be a sterile injection solution or suspension in a non-toxic parenterally acceptable diluent or solvent, for example, a solution in 1,3-butanediol. Acceptable carriers and solvents that can be used include water, Ringer's solution, and isotonic sodium chloride solution. In addition, sterile fixed oils are conventionally used as solvents or suspension media. For this reason, any mild fixed oil can be used, including synthetic mono- or di-glycerides. Also, fatty acids such as oleic acid can be used as the injectable formulations. Carrier formulations suitable for oral, subcutaneous, intravenous, intramuscular, etc. administration can be found in Remington's Pharmaceutical Sciences, Mack Publishing Co., Easton, PA.

The fusion protein or conjugate of the present invention can be prepared with carriers that protect it from rapid release, such as a controlled release formulation, including implants, transdermal patches, and microencapsulated delivery systems. Biodegradable and biocompatible polymers can be used, such as ethylene vinyl acetate, polyanhydrides, polyglycolic acid, collagen, polyorthoesters, and polylactic acid. Many methods for preparing such formulations are known in the prior art, see, for example, Sustained and Controlled Release Drug Delivery Systems, J.R. Robinson, ed., Marcel Dekker, Inc., New York, 1978, etc.

The pharmaceutical composition of the present invention can be administered by any conventional route, including injection or gradual infusion over time. For example, the administration can be oral, intravenous, intraperitoneal, intramuscular, intracavity, intratumor, or transdermal.

The pharmaceutical composition of the present invention is administered in an effective amount. An "effective amount" is the amount of any fusion protein or conjugate provided herein, alone or with further doses and/or other therapeutic agents, producing a desired response (e.g., reducing blood glucose levels in subjects). This may include only temporarily slowing the development of diabetes, or in some embodiments, permanently stopping the development of diabetes.

Of course, such an amount will depend on the specific disease to be treated, the severity of the disease, individual patient parameters (including age, physical condition, height and body weight), duration of treatment, the nature of the concurrent treatment (if any), the specific route of administration, and similar factors within the knowledge of medical and health workers. These factors are well known to those skilled in the art, and can be known only by using routine experiments. It is generally preferred to use the maximum dose of each component or combination thereof, that is to say the highest safe dose based on reasonable medical judgment. However, those skilled in the art can understand that patients may require lower doses or allowable doses for medical, psychological, or basically any other reasons.

The pharmaceutical composition used in the foregoing method is preferably sterile and contains an effective amount of the fusion protein or conjugate alone or in combination with another preparation in a weight unit or volume unit suitable for administration to patients to produce the desired response, such as a decrease in blood glucose.

The dosage of the fusion protein or conjugate administered to the subject can be selected according to different parameters, especially according to the mode of administration and the state of the subject. Other factors include the required treatment period. If the response in the subject at the initial dose applied is insufficient, a higher dose (or an effective higher dose achieved by a different, more localized delivery route) within the patient's tolerance can be applied.

In some embodiments, the pharmaceutical composition of the present invention contains 0.20 mg/ml ~ 5 mg/ml GLP-1 fusion protein and/or 4 mg/ml ~ 40 mg/ml GLP-1 fusion protein conjugate, preferably 0.20 mg/ml ~ 5 mg/ml GLP-1 fusion protein and/or 4 mg/ml ~ 40 mg/ml GLP-1 fusion protein conjugate, more preferably 0.5 mg/ml ~ 2 mg/ml GLP-1 fusion protein and/or 10 mg/ml ~ 20 mg/ml GLP-1 fusion protein conjugate. Generally, the dosage of the GLP-1 fusion protein or GLP-1 fusion protein conjugate of the present invention can range from about 10 µg/kg of the patient's body weight to about 100,000 µg/kg of the patient's body weight. In some embodiments, the dosage may range from about 0.1 mg/kg to about 20 mg/kg. In other embodiments, the dosage may range from about 0.1 mg/kg to 5 mg/kg, 0.1 mg/kg to 10 mg/kg, or 0.1 mg/kg to 15 mg/kg. In other embodiments, the dosage may range from about 1 mg/kg to 5 mg/kg, 5 mg/kg to 10 mg/kg, 10 mg/kg to 15 mg/kg, or 15 mg/kg to 20 mg/kg. In other embodiments, the dosage is about 0.1 mg/kg, 0.5 mg/kg, 1 mg/kg, 2 mg/kg, 3 mg/kg, 5 mg/kg, 7 mg/kg, 10 mg/kg, 12 mg/kg, 15 mg/kg, 17 mg/kg, 20 mg/kg, 25 mg/kg, or 30 mg/kg. In another embodiment, the dosage is about 1 mg/kg, 3 mg/kg, 5 mg/kg, or 6 mg/kg. Based on the property of the composition, the dose can be delivered continuously (for example, by a continuous pump) or at periodic intervals. In some embodiments, when administered intravenously, the dosage of the GLP-1 fusion protein or GLP-1 fusion protein conjugate of the present invention may be 0.1 to 20 mg/kg or any value therein. The ideal time interval for multiple administrations of a particular composition can be determined by those skilled in the art without undue experimentation. Other dosage regimens of the provided composition are known to those skilled in the art, where the dosage, administration schedule, administration site, administration mode, etc. may be different from the foregoing. In one embodiment, the dosage is administered intravenously. In another embodiment, the dosage regimen is a single intravenous administration.

A kit comprising the GLP-1 fusion protein or GLP-1 fusion protein conjugate (for example in the pharmaceutical composition) and instructions for use are also within the scope of the present invention. The kit may additionally contain at least one of other agents, such as one or more of other agents reducing blood glucose. In another embodiment, the kit may include a carrier that is compartmentalized to tightly hold one or more container devices or a series of container devices (such as test tubes, tubes, flasks, bottles, syringes, etc.). The components of the kit can be packaged in an aqueous medium or in a lyophilized form.

The composition may be provided in a lyophilized form or in an aqueous medium.

Preferably, the subject is a vertebrate, more preferably a mammal, most preferably human, but the subject can also be other animals, such as domestic animals (e.g., dogs, cats, etc.), livestock (e.g., cattle, sheep and goats, pigs, horses, etc.) or experimental animals (e.g., monkeys, rats, mice, rabbits, guinea pigs, etc.).

The GLP-1 fusion protein and/or GLP-1 fusion protein conjugate of the present invention may be administered alone, but is preferably administered as a pharmaceutical composition, which usually contains suitable pharmaceutical excipients, diluents or carriers selected according to the planned route of administration and it can be applied to the patient/subject in need of treatment in any suitable way. The precise dosage will depend on a number of factors, including the precise nature of the GLP-1 fusion protein and GLP-1 fusion protein conjugate.

Some suitable ways of administration include (without limitation) oral, rectal, nasal, topical (including buccal and sublingual), transdermal, vaginal or parenteral (including subcutaneous, intramuscular, intravenous, intradermal, intrathecal and epidural) administration.

In some embodiments, the pharmaceutical composition of the present invention contains an isotonic modifier and/or a preservative, preferably the isotonic modifier is one or more of sucrose, mannitol, sodium chloride and glycerol, and the preservative is selected from m-cresol, benzyl alcohol, methyl p-hydroxybenzoate, ethyl p-hydroxybenzoate, propyl p-hydroxybenzoate and butyl p-hydroxybenzoate. Those skilled in the art are able to prepare solutions suitable for the GLP-1 fusion protein or GLP-1 fusion protein of the present invention by using, for example, isotonic excipients such as physiological saline, Ringer's injection and lactated Ringer's injection. According to requirements, stabilizers, buffers, antioxidants and/or other additives can also be added. The pharmaceutical composition for oral administration can be in the form of tablets, capsules, powders or oral liquids. Tablets may include solid carriers such as gelatin or adjuvants. Liquid pharmaceutical compositions usually include a liquid carrier, such as water, petroleum, animal or vegetable oil, mineral oil, or synthetic oil, and it may also include physiological saline solution, glucose or other sugar solutions or glycols, such as ethylene glycol, propylene glycol or polyethylene glycol. In some embodiments, the pharmaceutical composition is in the form of a liquid preparation and/or a lyophilized preparation. Preferably the lyophilized preparation contains a lyoprotectant, and more preferably the lyoprotectant is selected from sucrose, lactose, mannitol, trehalose and other sugars.

The GLP-1 fusion protein and/or GLP-1 fusion protein conjugate described herein is preferably administered to a subject in a "therapeutically effective amount" or "effective amount". The composition is preferably administered to the subject in a "therapeutically effective amount", and the therapeutically effective amount or effective amount is sufficient to show its benefit to the subject. The actual amount administered, as well as the rate and time course of administration, will depend on the condition and severity of the subject to be treated. The prescription of treatment (such as determining the dosage) is determined by the medical staff, and usually the disease to be treated, the condition of the individual patient, the delivery site, the route of administration, and other factors known to doctors are under consideration.

In some embodiments, the dosage range of the GLP-1 fusion protein and/or GLP-1 fusion protein conjugate may be 30 mg/kg of body weight/day to 0.00001 mg/kg of body weight/day, 3 mg/kg/day to 0.0001 mg/kg/day, or 0.3 mg/kg/day to 0.01 mg/kg/day.

The present invention also provides a method for the treatment of diseases, comprising administering a therapeutically effective amount of the GLP-1 fusion protein and/or GLP-1 fusion protein conjugate to a subject in need thereof. In some embodiments, the disease is selected from the group consisting of: postprandial dumping syndrome, postprandial hyperglycemia, impaired glucose tolerance, obesity, eating disorders, insulin resistance syndrome, diabetes, and hyperglycemia. In a preferred embodiment, the disease is type II diabetes.

The present invention will be further illustrated by the following examples, which should not be construed as further limitations in any way. In the following examples, if not specifically noted, the reagents and materials used are commercially available products with at least analytical purity or equivalent levels.

## EXAMPLES

Example 1 Preparation of GLP-1 fusion protein

1. Construction of eukaryotic expression vector of GLP-1 fusion protein

The GLP-1 fusion protein constructed in this example has a structure of GLP-1-L1-Fc-L2-ST, wherein GLP-1 represents various forms of GLP-1 or analogs thereof, preferably the sequence of GLP-1 is with the same as dulaglutide (Trulicity™). L1 and L2 represent the first and second linkers respectively, wherein the sequence of L1 is GGGGSGGGGSGGGGSA, and the sequence of L2 is GGGGSGGGGS, GGGGSGGGGSGGGGS, or GSGGGSGGGGSGGGGS, or there is no L2. Fc represents the immunoglobulin Fc region, which is derived from human IgG2 (also representing as Fc2 in the present invention) or IgG4 (also representing as Fc4 in the present invention).Preferably, the Fc region is the same as dulaglutide (Trulicity™) which is a variant of human IgG4 (Fc4). ST represents sortase A recognition site with the ST sequence of LPETG or LPETGGG.

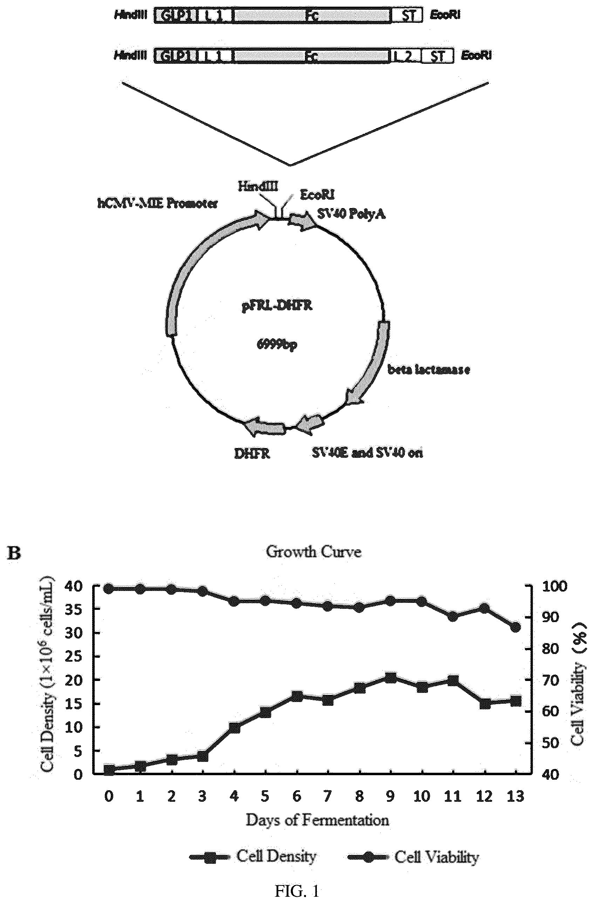

The amino acid sequences of different GLP-1 fusion proteins were reverse-translated into nucleotide sequences according to the codon preference of CHO cells. The full-length DNA fragment of GLP-1 fusion protein was obtained by whole gene synthesis and PCR amplification, and then cloned into the eukaryotic expression vector pFRL-DHFR through HindIII-EcoRI restriction sites at both ends. The vector pFRL-DHFR contained the human cytomegalovirus promoter (hCMV-MIE Promoter), SV40 PolyA, DHFR (dihydrofolate reductase) and other elements, which can assist in the efficient and stable expression of foreign genes in eukaryotic cells (FIG. 1A).

Table 1 List of different GLP-1 fusion proteins constructed in the Example

Table 1. Amino acid sequence characteristics of different GLP-1 fusion proteins

| Protein name | GLP-1 | L1 | Fc | L2 | ST |
|---|---|---|---|---|---|
| GLP1-L1-Fc4-ST | Consistent with dulaglutide | GGGGSGGG GSGGGGSA | Human IgG4, consistent with dulaglutide | No | LPETG |
| GLP1-L1-Fc4-L2-ST | Consistent with dulaglutide | GGGGSGGG GSGGGGSA | Human IgG4, consistent with dulaglutide | GGGGSGG GGSGGGG S | LPETG |
| GLP1-L1-Fc2-L2-ST | Consistent with dulaglutide | GGGGSGGG GSGGGGSA | Human IgG2 | GSGGGSG GGGSGGG GS | LPETG |

(continued)

| Protein name | GLP-1 | L1 | Fc | L2 | ST |
|---|---|---|---|---|---|
| GLP1-L1-Fc2-ST | Consistent with dulaglutide | GGGGSGGG GSGGGGSA | Human IgG2 | No | LPETG |
| GLP1-L1-Fc4-L2-STG3 | Consistent with dulaglutide | GGGGSGGG GSGGGGSA | Human IgG4, consistent with dulaglutide | GGGGSGG GGS | LPETGG G |

1. Amino acid sequence of GLP1-L1-Fc4-ST

HGEGTFTSDVSSYLEEQAAKEFIAWLVKGGGGGGGSGGGGSGGGGSAESKYGP
PCPPCPAPEAAGGPSVFLFPPKPKDTLMISRTPEVTCVVVDVSQEDPEVQFNWYVDGV
EVHNAKTKPREEQFNSTYRVVSVLTVLHQDWLNGKEYKCKVSNKGLPSSIEKTISKA
KGQPREPQVYTLPPSQEEMTKNQVSLTCLVKGFYPSDIAVEWESNGQPENNYKTTPPV
LDSDGSFFLYSRLTVDKSRWQEGNVFSCSVMHEALHNHYTQKSLSLSLGLPETG (SEQ
ID NO: 1)

Nucleotide sequence of GLP1-L1-Fc4-ST:

CACGGCGAGGGCACTTTCACATCCGACGTGTCTAGCTACTTGGAAGAGCAGG
CCGCTAAGGAGTTCATCGCTTGGCTCGTGAAGGGAGGTGGTGGTGGAGGTGGATC
TGGCGGAGGAGGATCTGGAGGTGGAGGAAGCGCCGAGTCCAAGTACGGACCTCC
ATGCCCACCTTGCCCAGCACCAGAGGCCGCTGGCGGCCCATCTGTGTTCCTGTTCC
CACCAAAGCCAAAGGACACACTCATGATTAGCCGGACCCCTGAGGTGACATGCGT
GGTGGTGGACGTGTCTCAGGAGGACCCCGAGGTGCAGTTCAACTGGTACGTGGAC
GGCGTGGAGGTGCACAACGCTAAGACCAAGCCACGCGAGGAGCAGTTCAACAGC
ACATACCGGGTGGTGAGCGTGCTGACTGTGCTGCACCAGGACTGGCTGAACGGCA
AGGAGTACAAGTGCAAGGTGTCTAACAAGGGCCTGCCTAGCTCTATTGAAAAGAC
TATTTCTAAGGCTAAGGGCCAGCCTAGAGAGCCTCAGGTGTACACACTGCCACCTT
CTCAGGAGGAGATGACAAAGAACCAGGTGTCCCTGACTTGCCTCGTGAAGGGCTT

CTACCCATCCGACATCGCTGTGGAGTGGGAGAGCAACGGCCAGCCCGAGAACAAC
TACAAGACTACTCCTCCAGTGCTCGACAGCGACGGCAGCTTCTTCCTGTACTCCAG
ACTCACAGTGGACAAGTCCCGTTGGCAGGAGGGCAACGTGTTCTCTTGCTCCGTG
ATGCACGAGGCCCTCCACAACCACTACACTCAGAAGTCCCTGTCTCTGTCCCTGG
GCCTGCCCGAAACTGGG (SEQ ID NO: 2)

2. Amino acid sequence of GLP1-L1-Fc4-L2-ST

HGEGTFTSDVSSYLEEQAAKEFIAWLVKGGGGGGGSGGGGSGGGGSAESKYGP
PCPPCPAPEAAGGPSVFLFPPKPKDTLMISRTPEVTCVVVDVSQEDPEVQFNWYVDGV
EVHNAKTKPREEQFNSTYRVVSVLTVLHQDWLNGKEYKCKVSNKGLPSSIEKTISKA
KGQPREPQVYTLPPSQEEMTKNQVSLTCLVKGFYPSDIAVEWESNGQPENNYKTTPPV
LDSDGSFFLYSRLTVDKSRWQEGNVFSCSVMHEALHNHYTQKSLSLSLGGGGGSGGG
GSGGGGSLPETG (SEQ ID NO: 3)

Nucleotide sequence of GLP1-L1-Fc4-L2-ST:

CACGGCGAGGGCACTTTCACATCCGACGTGTCTAGCTACTTGGAAGAGCAGG
CCGCTAAGGAGTTCATCGCTTGGCTCGTGAAGGGAGGTGGTGGTGGAGGTGGATC
TGGCGGAGGAGGATCTGGAGGTGGAGGAAGCGCCGAGTCCAAGTACGGACCTCC
ATGCCCACCTTGCCCAGCACCAGAGGCCGCTGGCGGCCCATCTGTGTTCCTGTTCC
CACCAAAGCCAAAGGACACACTCATGATTAGCCGGACCCCTGAGGTGACATGCGT
GGTGGTGGACGTGTCTCAGGAGGACCCCGAGGTGCAGTTCAACTGGTACGTGGAC
GGCGTGGAGGTGCACAACGCTAAGACCAAGCCACGCGAGGAGCAGTTCAACAGC
ACATACCGGGTGGTGAGCGTGCTGACTGTGCTGCACCAGGACTGGCTGAACGGCA
AGGAGTACAAGTGCAAGGTGTCTAACAAGGGCCTGCCTAGCTCTATTGAAAAGAC
TATTTCTAAGGCTAAGGGCCAGCCTAGAGAGCCTCAGGTGTACACACTGCCACCTT
CTCAGGAGGAGATGACAAAGAACCAGGTGTCCCTGACTTGCCTCGTGAAGGGCTT
CTACCCATCCGACATCGCTGTGGAGTGGGAGAGCAACGGCCAGCCCGAGAACAAC
TACAAGACTACTCCTCCAGTGCTCGACAGCGACGGCAGCTTCTTCCTGTACTCCAG
ACTCACAGTGGACAAGTCCCGTTGGCAGGAGGGCAACGTGTTCTCTTGCTCCGTG
ATGCACGAGGCCCTCCACAACCACTACACTCAGAAGTCCCTGTCTCTGTCCCTGG
GCGGAGGTGGCGGCTCTGGAGGCGGAGGTAGTGGTGGCGGCGGTTCACTGCCCG
AAACTGGG (SEQ ID NO: 4)

3. Amino acid sequence of GLP1-L1-Fc2-L2-ST

HGEGTFTSDVSSYLEEQAAKEFIAWLVKGGGGGGGSGGGGSGGGGSAVECPPCP

APPVAGPSVFLFPPKPKDTLMISRTPEVTCVVVDVSHEDPEVQFNWYVDGVEVHNAK
TKPREEQFNSTFRVVSVLTVVHQDWLNGKEYKCKVSNKGLPAPIEKTISKTKGQPREP
QVYTLPPSREEMTKNQVSLTCLVKGFYPSDIAVEWESNGQPENNYKTTPPMLDSDGSF
FLYSKLTVDKSRWQQGNVFSCSVMHEALHNHYTQKSLSLSPGGSGGGSGGGGSGGG
GSLPETG (SEQ ID NO: 5)

Nucleotide sequence of GLP1-L1-Fc2-L2-ST:

CACGGCGAGGGAACATTCACCAGCGACGTGTCCAGCTACCTGGAGGAGCAG
GCCGCCAAGGAGTTTATCGCCTGGCTCGTGAAAGGAGGCGGCGGCGGAGGAGGT
TCTGGCGGCGGCGGCTCCGGCGGAGGCGGATCCGCTGTTGAATGTCCTCCATGTCC
AGCTCCACCAGTTGCTGGGCCTTCCGTCTTCCTATTCCCCCCAAAGCCTAAGGACA
CCCTGATGATATCTCGTACCCCTGAGGTGACCTGTGTCGTAGTGGATGTGAGTCATG
AAGATCCAGAAGTGCAGTTCAACTGGTATGTGGATGGTGTCGAAGTGCATAATGCT
AAGACCAAGCCACGAGAGGAGCAGTTTAACTCAACCTTTAGGGTTGTGAGTGTGT
TGACTGTCGTCCATCAAGATTGGTTAAACGGTAAAGAATACAAGTGTAAAGTCTCC
AACAAGGGATTGCCTGCCCCTATTGAGAAGACCATTTCCAAGACGAAAGGCCAAC
CACGTGAGCCTCAAGTGTATACTCTACCCCCAAGTCGAGAGGAGATGACTAAGAAT
CAAGTCTCACTTACGTGTCTTGTCAAAGGTTTCTACCCATCAGACATTGCCGTGGA
GTGGGAGTCAAATGGTCAACCCGAGAATAATTATAAGACCACTCCCCCCATGTTAG
ACTCAGACGGTTCTTTCTTCTTGTACTCTAAACTTACTGTCGACAAATCCCGATGGC
AACAAGGCAATGTGTTCTCCTGTTCCGTCATGCACGAGGCCTTACACAATCACTAT
ACCCAGAAATCCTTGTCTTTGTCTCCAGGGGGTTCTGGCGGTGGCTCCGGTGGAG
GCGGAAGCGGCGGTGGAGGATCACTGCCCGAAACTGGG (SEQ ID NO: 6)

4. Amino acid sequence of GLP1-L1-Fc2-ST

HGEGTFTSDVSSYLEEQAAKEFIAWLVKGGGGGGGSGGGGSGGGGSAVECPPCP APPVAGPSVFLFPPKPKDTLMISRTPEVTCVVVDVSHEDPEVQFNWYVDGVEVHNAK TKPREEQFNSTFRVVSVLTVVHQDWLNGKEYKCKVSNKGLPAPIEKTISKTKGQPREP QVYTLPPSREEMTKNQVSLTCLVKGFYPSDIAVEWESNGQPENNYKTTPPMLDSDGSF FLYSKLTVDKSRWQQGNVFSCSVMHEALHNHYTQKSLSLSPGKLPETG (SEQ ID NO: 7)

Nucleotide sequence of GLP1-L1-Fc2-ST:

CACGGCGAGGGAACATTCACCAGCGACGTGTCCAGCTACCTGGAGGAGCAG GCCGCCAAGGAGTTTATCGCCTGGCTCGTGAAAGGAGGCGGCGGCGGAGGAGGT

TCTGGCGGCGGCGGCTCCGGCGGAGGCGGATCCGCTGTTGAATGTCCTCCATGTCC AGCTCCACCAGTTGCTGGGCCTTCCGTCTTCCTATTCCCCCCAAAGCCTAAGGACA CCCTGATGATATCTCGTACCCCTGAGGTGACCTGTGTCGTAGTGGATGTGAGTCATG AAGATCCAGAAGTGCAGTTCAACTGGTATGTGGATGGTGTCGAAGTGCATAATGCT AAGACCAAGCCACGAGAGGAGCAGTTTAACTCAACCTTTAGGGTTGTGAGTGTGT TGACTGTCGTCCATCAAGATTGGTTAAACGGTAAAGAATACAAGTGTAAAGTCTCC AACAAGGGATTGCCTGCCCCTATTGAGAAGACCATTTCCAAGACGAAAGGCCAAC CACGTGAGCCTCAAGTGTATACTCTACCCCCAAGTCGAGAGGAGATGACTAAGAAT CAAGTCTCACTTACGTGTCTTGTCAAAGGTTTCTACCCATCAGACATTGCCGTGGA GTGGGAGTCAAATGGTCAACCCGAGAATAATTATAAGACCACTCCCCCCATGTTAG ACTCAGACGGTTCTTTCTTCTTGTACTCTAAACTTACTGTCGACAAATCCCGATGGC AACAAGGCAATGTGTTCTCCTGTTCCGTCATGCACGAGGCCTTACACAATCACTAT ACCCAGAAATCCTTGTCTTTGTCTCCAGGGAAACTGCCCGAACTGGG (SEQ ID NO: 8)

5. Amino acid sequence of GLP1-L1-Fc4-L2-STG3:

HGEGTFTSDVSSYLEEQAAKEFIAWLVKGGGGGGGSGGGGSGGGGSAESKYGP
PCPPCPAPEAAGGPSVFLFPPKPKDTLMISRTPEVTCVVVDVSQEDPEVQFNWYVDGV
EVHNAKTKPREEQFNSTYRVVSVLTVLHQDWLNGKEYKCKVSNKGLPSSIEKTISKA
KGQPREPQVYTLPPSQEEMTKNQVSLTCLVKGFYPSDIAVEWESNGQPENNYKTTPPV
LDSDGSFFLYSRLTVDKSRWQEGNVFSCSVMHEALHNHYTQKSLSLSLGGGGGSGGG
GSLPETGGG (SEQ ID NO: 22)

Nucleotide sequence of GLP1-L1-Fc4-L2-STG3:

CACGGCGAGGGCACTTTCACATCCGACGTGTCTAGCTACTTGGAAGAGCAGGCCG
CTAAGGAGTTCATCGCTTGGCTCGTGAAGGGAGGTGGTGGTGGAGGTGGATCTGG
CGGAGGAGGATCTGGAGGTGGAGGAAGCGCCGAGTCCAAGTACGGACCTCCATG
CCCACCTTGCCCAGCACCAGAGGCCGCTGGCGGCCCATCTGTGTTCCTGTTCCCAC
CAAAGCCAAAGGACACACTCATGATTAGCCGGACCCCTGAGGTGACATGCGTGGT
GGTGGACGTGTCTCAGGAGGACCCCGAGGTGCAGTTCAACTGGTACGTGGACGG
CGTGGAGGTGCACAACGCTAAGACCAAGCCACGCGAGGAGCAGTTCAACAGCAC
ATACCGGGTGGTGAGCGTGCTGACTGTGCTGCACCAGGACTGGCTGAACGGCAAG
GAGTACAAGTGCAAGGTGTCTAACAAGGGCCTGCCTAGCTCTATTGAAAAGACTAT
TTCTAAGGCTAAGGGCCAGCCTAGAGAGCCTCAGGTGTACACACTGCCACCTTCTC
AGGAGGAGATGACAAAGAACCAGGTGTCCCTGACTTGCCTCGTGAAGGGCTTCTA

CCCATCCGACATCGCTGTGGAGTGGGAGAGCAACGGCCAGCCCGAGAACAACTAC
AAGACTACTCCTCCAGTGCTCGACAGCGACGGCAGCTTCTTCCTGTACTCCAGACT
CACAGTGGACAAGTCCCGTTGGCAGGAGGGCAACGTGTTCTCTTGCTCCGTGATG
CACGAGGCCCTCCACAACCACTACACTCAGAAGTCCCTGTCTCTGTCCCTGGGCG
GAGGAGGTGGATCTGGTGGAGGCGGATCTCTGCCCGAAACTGGGGGTGGA    (SEQ
ID NO: 23)

2. Screening and fermentation of stable cell lines

2.1 Screening of stable cell lines

Each plasmid constructed above was transfected into the host cell CHO DG44 by electrotransfection with the cell density of $6 \times 10^6$ cells/mL, the volume of 0.8mL, and the plasmid amount of 40μg. The plasmid and cells were gently mixed and then added to the electroporation cuvettes (Bio-Rad, 4mm). The electroporator used here was from Bio-Rad (GENE PULSER XCELL) and the parameters of electroporation were as follows: voltage 290V and pulse duration 20 ms.

After electroporation, the cells were transferred to petri dishes containing 15 mL of recovery medium for 48 hours of culture, and then inoculated into 96-well plates with screening medium containing 50 nM MTX. When the cell confluence reached more than 50%, cell lines were screened for high expression by dot blotting method and the antibody used was goat anti-human IgG Fc. The selected clones with relatively high expression levels were sequentially transferred to 24-well plates, 6-well plates, T25 cell culture flasks and cell culture shake flasks for expansion.

In order to increase the yield of fusion protein, the cells were cultured under pressure with increasing MTX concentration. Through the inhibition of DHFR gene by MTX, the co-amplification of DHFR gene and fusion protein gene was realized.

2.2 Fermentation

The cells were inoculated to a volume of 8L and a density of $0.8\text{-}1.6 \times 10^6$ cells/mL into a 15L bioreactor (Applikon, Biobundle 15L). The culture parameters were set as follows: pH $6.95 \pm 0.15$, DO 45%, oxygen supply: large bubble ventilation, and rotation speed: 80-140rpm. When the cell density was about $10\text{-}12 \times 10^6$ cells/mL on the 5th day of culture, the culture temperature was lowered to 33°C. Feeding was started from the fourth day of culture, and the residual glucose content was measured every day. Sugar was supplemented to reach a concentration of 3 g/L every day before lowering the temperature, and 4 g/L after lowering the temperature. During the fermentation, the cell count and viability were measured every day. On day 13, the cells were harvested when the viability was about 90%.

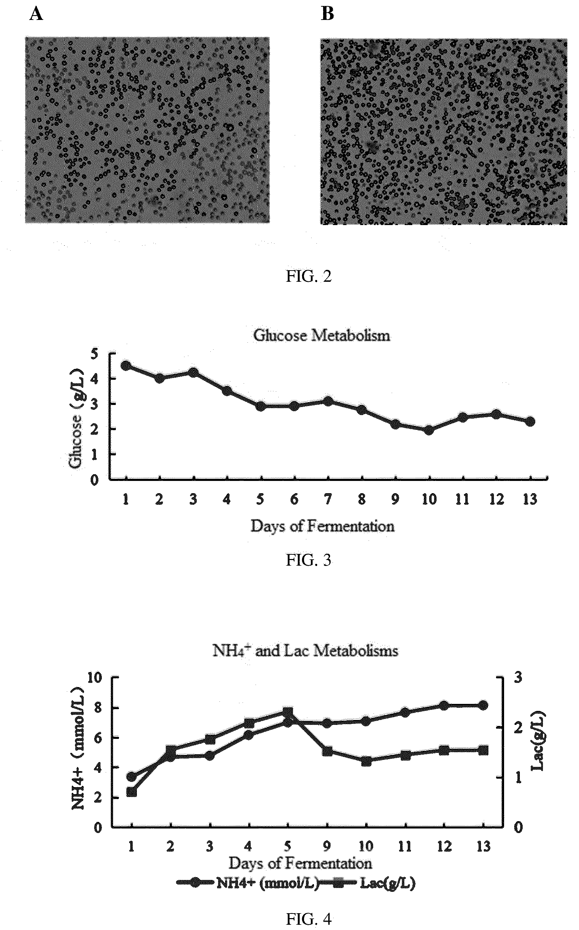

FIG. 1B to FIG. 4 show the growth curve, cell viability, microscopic examination results and various process parameters of the GLP1-L1-Fc2-L2-ST stable cell line (clone number: P1-3G3C2) during fermentation. It can be seen from FIG. 1B that on the ninth day of fermentation, the cell density was the highest with $20.5 \times 10^6$/mL, and on the 13th day when the cells were harvested, the cell density was $15.5 \times 10^6$/mL and the cell viability was 86.6%. The microscopic examinations show that the morphologies of cells were good without obvious dead cells (FIG. 2A and FIG. 2B). FIG. 3 and FIG. 4 show the metabolism of glucose, $NH_4^+$ and lactic acid (Lac) during fermentation.

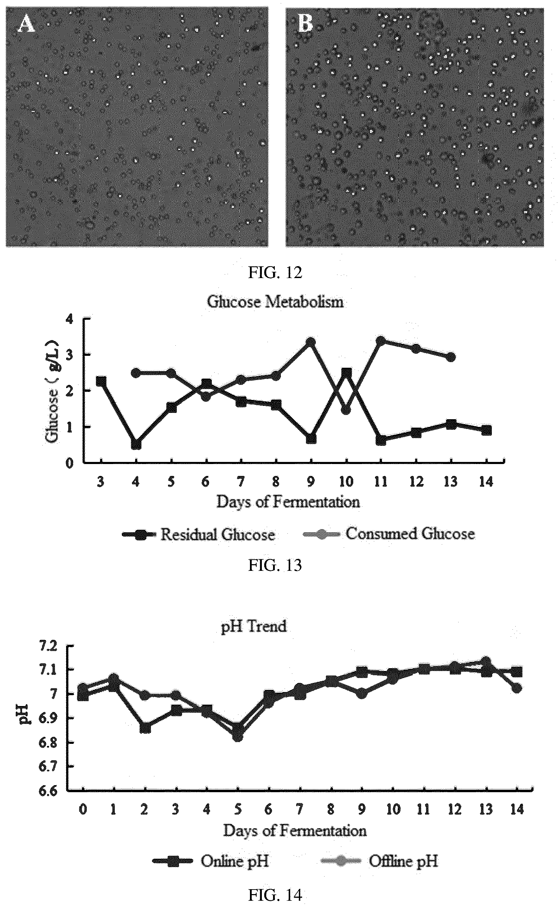

FIG. 11 to FIG. 14 show the growth curve, cell viability, microscopic examination results and various process parameters of GLP1-L1-Fc4-L2-STG3 during fed-batch fermentation. It can be seen from FIG. 11B that on the eighth day of fermentation, the cell density was the highest with $19.9 \times 10^6$ cells/mL, and on the 14th day when the cells were harvested, the cell density was $16.0 \times 10^6$ cells/mL and the cell viability was 92%. The microscopic examinations show that the morphologies of cells were good without obvious dead cells (FIG. 12A and FIG. 12B). FIG. 13 and FIG. 14 show the glucose metabolism and pH changes during fermentation.

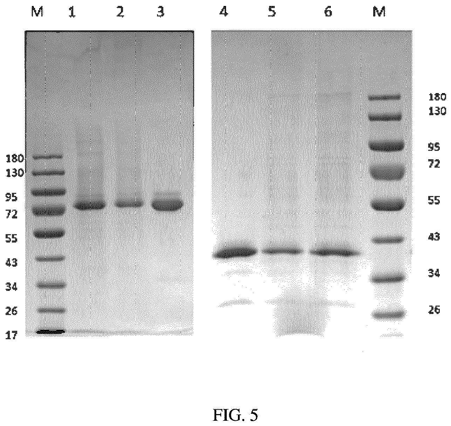

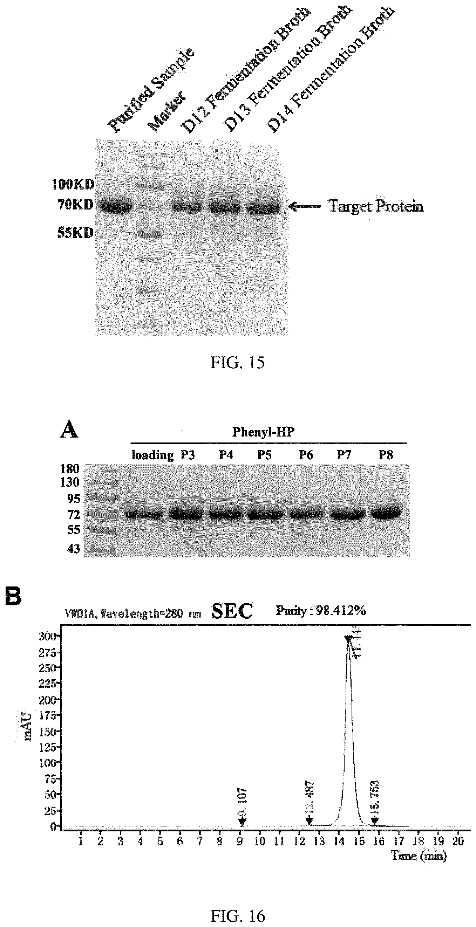

The content of GLP1-L1-Fc2-L2-ST protein in the fermentation supernatant was 1.8g/L and the content of GLP1-L1-Fc4-L2-STG3 was 1.19g/L by molecular interaction instrument (Pall Life Science, Qke). SDS-PAGE electrophoresis was used to detect the integrity of the GLP1-L1-Fc2-L2-ST protein. GLP1-L1-Fc2-L2-ST is a double-chain Fc fusion protein with a molecular weight of 61.6kDa (without sugar chains). As shown in FIG. 5, the molecular weight of GLP1-L1-Fc2-L2-ST was basically the same as the theoretical molecular weight in the non-reduced and reduced state. At the same time, SDS-PAGE electrophoresis was used to detect the changes of GLP1-L1-Fc4-L2-STG3 protein (FIG. 15). It can be seen that the yield of target protein increased significantly from the 12th day to 14th day of fermentation, and in the supernatant, the target protein was in the majority, with only a small amount of impurity remaining. The expressed protein was identified by ELISA, and the result was positive.

2.3 Purification and detection

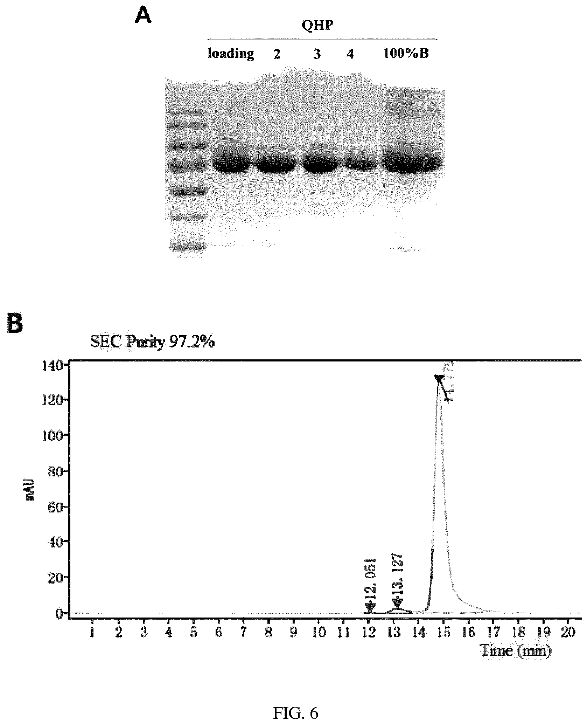

GLP1-L1-Fc2-L2-ST and GLP1-L1-Fc4-L2-STG3 in the supernatant of fermentation broth were purified in two steps: affinity chromatography (Uni mab, NanoMicro) and anion exchange chromatography (QHP, BXK). The purified protein was detected by SDS-PAGE and SEC (size exclusion chromatography). According to the results of SDS-PAGE (FIG. 6A and FIG. 16A), the size of the target protein was basically the same as the theoretical molecular weight. SEC was performed on Agilent Advancebio SEC 300A, 2.7$\mu$m, 7.8*300mm column, gradient elution was performed using 50 mM Tris+150 mM NaCl+10% acetonitrile, pH7.0 (column temperature: 30°C, flow rate: 0.5 mL/min, sample injector temperature: 4.0°C, sample injection volume: 50 $\mu$g, and sample analysis time: 35 min). It can be seen from FIG. 6B that the retention time of GLP1-L1-Fc2-L2-ST protein was 14.6 min, and the purity after two-step purification was 97.2%. It can be seen from FIG. 16B that the retention time of GLP1-L1-Fc4-L2-STG3 protein was 14.49 min, and the purity after two-step purification was 98.412%.

Example 2 Linking of 20kDa PEG and 40kDa PEG to GLP1-Fc fusion protein

The C-terminals of all the constructed GLP1-Fc fusion proteins contained sortase A core recognition sequence LPETG, which can be specifically recognized by sortase A. Under the action of sortase A, the amide bond between T and G was cut, and T reacted with the sulfhydryl group of C at sortase position 184 to form a thioester bond intermediate, which was then attacked by GGGAA-PEG with poly-Gly at the N-terminal. Finally, PEGs of different molecular weights were linked to the C-terminal of the protein.

The PEGylation reaction systems of GLP1-L1-Fc2-L2-ST and GLP1-L1-Fc2-ST proteins were as follows: 20kDa or 40kDa GGGAA-PEG (GGGAA was purchased from Shanghai GL Biochem Co., Ltd., and 20kDa or 40kDa GGGAA -PEG was synthesized by Beijing JenKem Technology Co., Ltd.) was dissolved in a buffer solution and adjusted to pH 8.0, and then the protein was added to the 50mM Tris 150mM NaCl pH8.0 buffer system at a feeding ratio of 1:15 and 1:10, respectively. Next, sortase A and 10 mM $CaCl_2$ were added. Sampling was performed at different time points of the reaction, and after 90 minutes, EDTA was added to stop the reaction.

The PEGylation reaction system of GLP1-L1-Fc4-L2-STG3 protein was as follows: 40kDa GGGAA-PEG (GGGAA was purchased from Shanghai GL Biochem Co., Ltd., and 40kDa GGGAA-PEG was synthesized by Beijing JenKem Technology Co., Ltd.) was dissolved in a buffer solution and adjusted to 7.0, and then the protein and PEG were added to the 20mM Tris 150mM NaCl pH7.0 buffer system at a feeding ratio of 1:15 (molar ratio). Next, sortase A and $CaCl_2$ (10mM) were added, 50 IU enzyme per mg of target protein, and the final concentration of the target protein was 6 mg/mL. After 30 minutes, EDTA was added to stop the reaction.

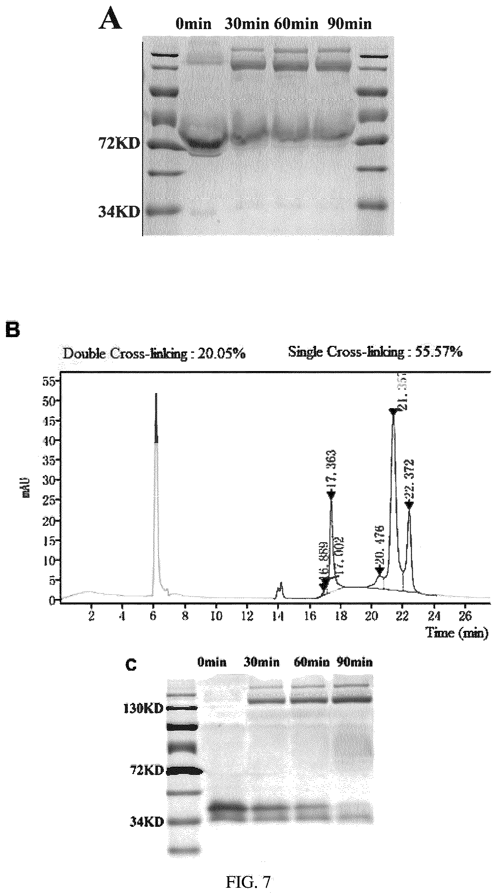

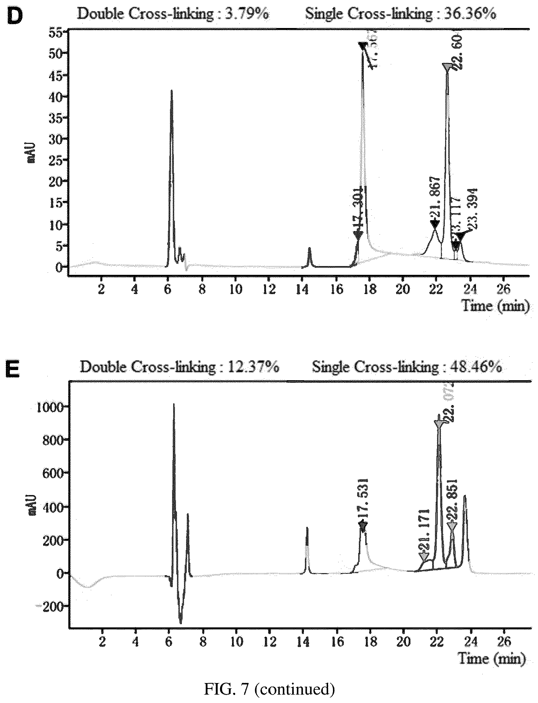

The above-mentioned products after linking were detected by SDS-PAGE and reversed phase chromatography (RPC) to detect the linkage rate (crosslinking rate). Single crosslinking means that only one monomer of the fusion protein dimer is linked to PEG, and double crosslinking means that both monomers of the fusion protein dimer are linked to PEG. Reversed phase chromatography test was performed on Waters Xbridge@protein BEH C4, 3.5 μm, 4.6*250mm chromatographic column with gradient elution with 0.1% TFA water and 0.1% TFA acetonitrile, at conditions: column temperature of 50°C, detection wavelength of 280 nm, flow rate of 0.5 mL/min, sample injector temperature of 4.0°C, sample injection volume of 20 μg and sample analysis time of 50 min. The test results of GLP1-L1-Fc2-L2-ST and GLP1-L1-Fc2-ST proteins are shown in FIG. 7. As the reaction time increased, the PEGylation products with 20kDa or 40kDa PEG gradually increased. At 90 min, the PEGylation products reached the maximum and the termination reaction was proceeded, and at this time, the total crosslinking rate of GLP1-L1-Fc2-L2-ST protein with 20kDa PEG was 75.62%, of which single crosslinking accounted for 55.57% and double crosslinking accounted for 20.05%. The total crosslinking rate of GLP1-L1-Fc2-L2-ST protein with 40kDa PEG was 40.15%, of which single crosslinking made up 36.36% and double crosslinking made up 3.79%. The total crosslinking rate of GLP1-L1-Fc2-ST protein with 40kDa PEG was 60.83%, of which single crosslinking rate was 48.46% and double crosslinking rate was 12.37%. It can be seen from the 40kDa PEG crosslinking results that, under the same reaction conditions, the crosslinking efficiency of GLP1-L1-Fc2-ST was much higher than that of GLP1-L1-Fc2-L2-ST.

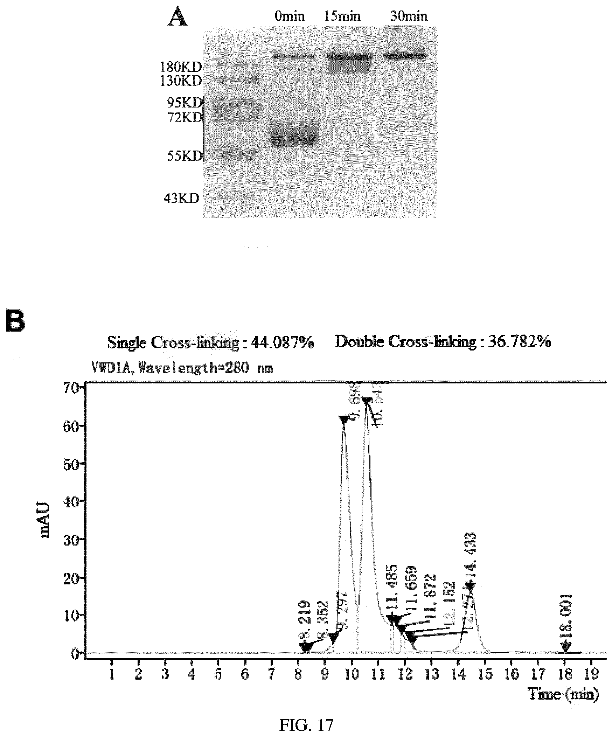

The test results of GLP1-L1-Fc4-L2-STG3 protein are shown in FIG. 17. As the reaction time increased, the 40kDa PEGylation product gradually increased. At 30min, the PEGylation product reached the maximum and the reaction was terminated, and at this time, the total crosslinking rate of GLP1-L1-Fc4-L2-STG3 protein with 40kDaPEG was 80.86%, of which single crosslinking accounted for 44.087% with peak time of 10.543min and double crosslinking accounted for 36.782% with peak time of 9.698min.

Purification and detection after crosslinking

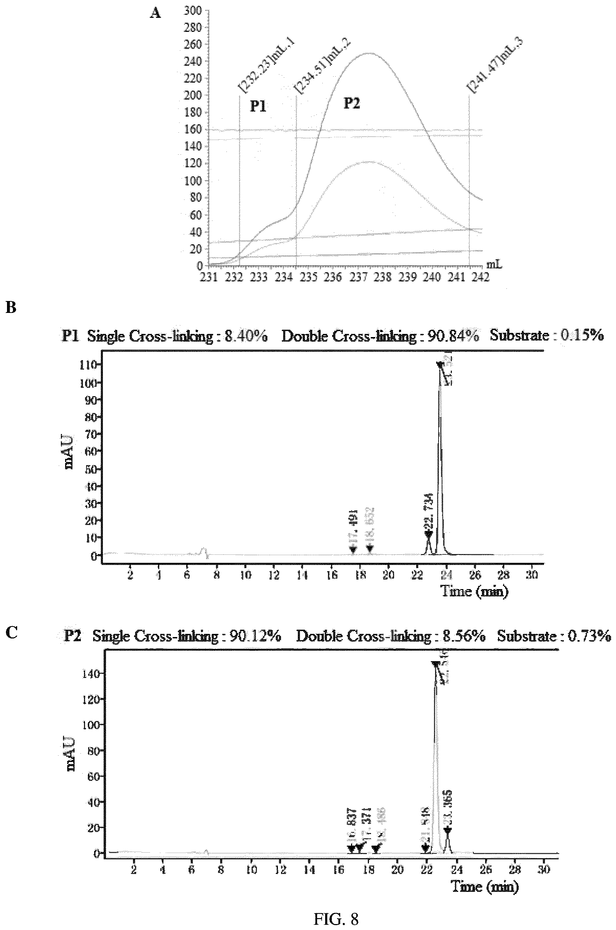

After crosslinking with 40kDa PEG, GLP1-L1-Fc2-L2-ST was subjected to Butyl HP hydrophobic chromatography to remove unreacted GGGAA-PEG and sortase A, and subjected to anion exchange chromatography to remove non-crosslinked substrate protein to control the residual amount of substrate within 1%. As shown in FIG. 8A, GLP1-L1-Fc2-L2-ST crosslinked with 40kDa PEG can be divided into two peaks P1 and P2 by anion exchange chromatography. RPC test results show (FIG. 8B) that the P1 peak was mainly double crosslinked products (double crosslinking product 90.84%, single crosslinking product 8.4%, substrate 0.15%), and P2 peak was mainly single crosslinked products (double crosslinking product 8.56%, single crosslinking product 90.12%, substrate 0.73%). The retention time of non-crosslinked GLP1-L1-Fc2-L2-ST was 17.3min, and the retention times of single and double crosslinked products were 22.5 and 23.2min, respectively.

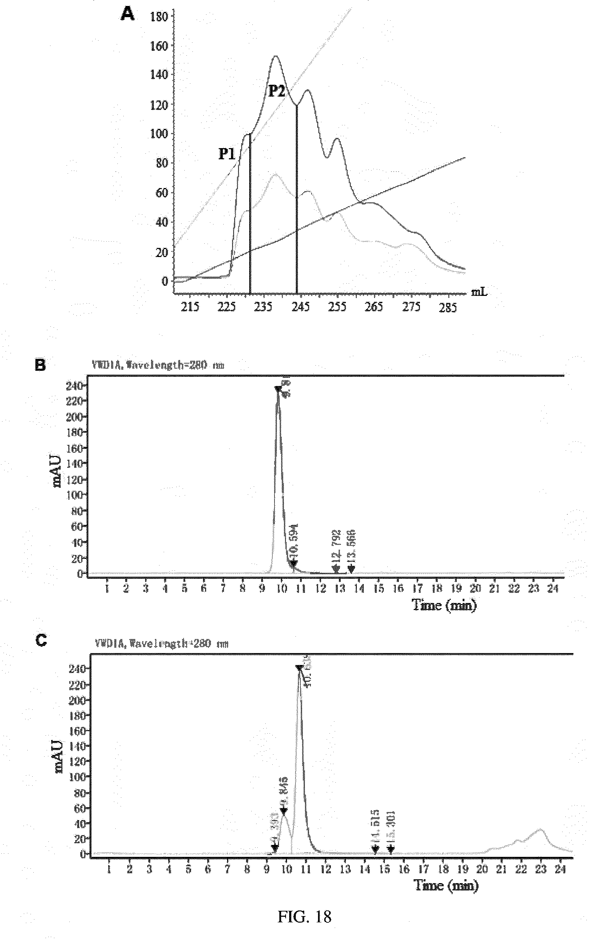

After crosslinking with 40kDa PEG, GLP1-L1-Fc4-L2-STG3 can be separated into single crosslinking, double crosslinking and substrates by anion exchange chromatography (SuperQ 5PW, TOSOH). The chromatogram is shown in FIG. 18A. According to the SEC test results (FIG. 18B and C), the P1 peak was mainly double crosslinked product (double crosslinking product 97.172%, single crosslinking product 2.766%, substrate 0%), and P2 peak was mainly single crosslinked product (double crosslinking product 19.722%, single crosslinking product 79.754%, substrate 0.158%). The retention times of single and double crosslinked products were 10.63 and 9.8 min, respectively.

Example 3 Activity detection of crosslinked GLP1-L1-Fc2-L2-ST protein

GLP-1 can activate adenylate cyclase (AC) by binding to GLP-1 receptor (GLP-1R) to produce cAMP, and cAMP further activates cAMP-dependent transcription factor, that is, cAMP response element binding protein (CREB), which activates the transcription of downstream genes after binding to cAMP response element (CRE).

The detection in this example included the following steps. HEK293/CRE-Luc/GLP1R cells (purchased from GenScript, #M00562) were cultured to the logarithmic growth phase, digested with trypsin and resuspended in complete medium to adjust the cell density to $1 \times 10^6$ cells/mL, and then the cells were added to white 96-well plates, 50μL/well.

Dulaglutide and the test substance were diluted with complete medium to 400 ng/mL, and further diluted at a 4-fold ratio with a total of 9 gradients, and then added to cells, 50$\mu$L/well. The cells were incubated at 37°C, 5% $CO_2$ for 5 h. Later, Bright-Glo Luciferase (promega) was add 100 $\mu$L per well and shielded from light for 10 min. A full-band fluorescence microplate reader (TECAN, 200PRO) was used to measure the fluorescence value.

Data were processed to fit a four-parameter equation by Softmax analysis software as the fluorescence values of the standard and test substances being the ordinate and the logarithmic value of the concentrations being the abscissa. The relative activity of the test substance was calculated according to the following equation:

$$\text{Relative activity of test substance} = \frac{EC_{50} \text{ of reference substance}}{EC_{50} \text{ of test substance}}$$

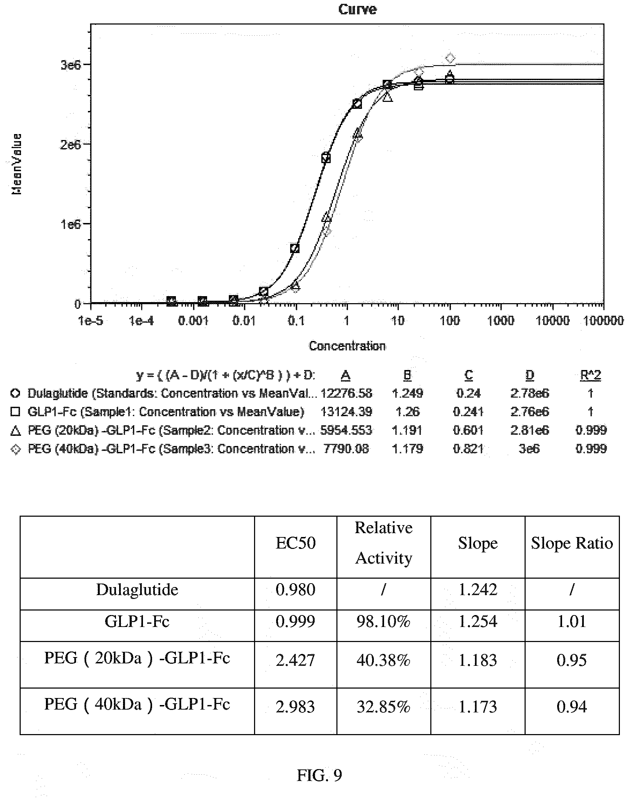

FIG. 9 shows the activity test results of GLP1-L1-Fc2-L2-ST crosslinked with 20kDa PEG and 40kDa PEG, respectively. Compared with dulaglutide, the relative activity of the non-crosslinked substrate (GLP1-Fc) was 98.10%. The activities of both PEG-crosslinked products decreased, wherein the relative activity of the product crosslinked with 20kDa PEG (indicated as PEG (20kDa)-GLP1-Fc in the figure) was 40.38% and the relative activity of 40kDa crosslinked product (indicated as PEG (40kDa)-GLP1-Fc in the figure) was 32.85%.

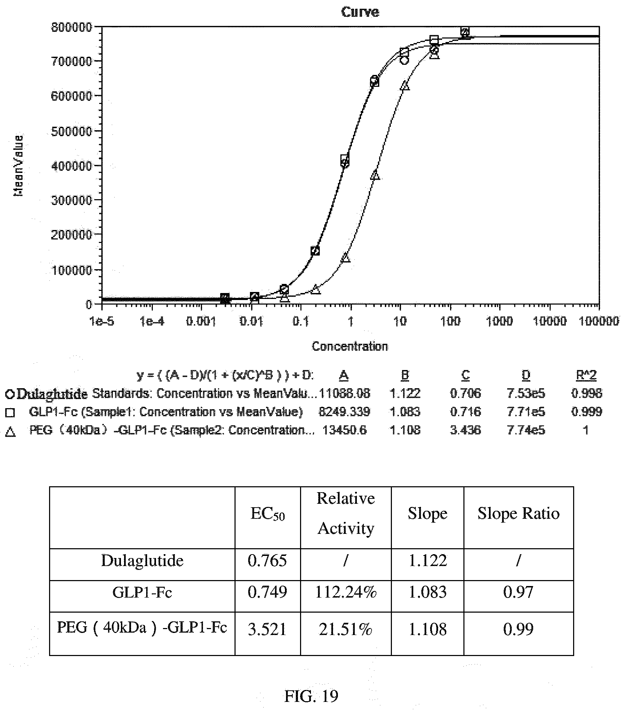

FIG. 19 shows the results of activity test of GLP1-L1-Fc4-L2-STG3 crosslinked with 40kDa PEG. Similar to the PEG-conjugated GLP1-L1-Fc2-L2-ST, compared with dulaglutide, the activity of 40kDa PEG-conjugated GLP1-L1-Fc4-L2-STG3 (indicated as PEG (40kDa)-GLP1-Fc) in the figure) was reduced, with the relative activity of 21.51%.

Example 4 *In vivo* hypoglycemic activity of GLP-1 fusion protein or PEG conjugate thereof

C57BL/6 mice (purchased from Beijing Vital River Co., Ltd.) were reared with 5 mice per cage, fed with normal mouse growth feed and free drinking water. The light time was 12h (08:00am-20:00pm) and the dark time was 12h (20:00pm-08:00am).

(1) Experimental results of GLP1-L1-Fc2-L2-ST

40 healthy male C57BL/6 mice aged 6-8 weeks were divided into 5 groups with 8 mice in each group. These groups include: (1) GLP1-Fc group (GLP-1-L1-Fc2-L2-ST substrate before crosslinking); (2) dulaglutide group; (3) 40kDa PEG GLP1-Fc group (GLP-1-L1-Fc2-L2-ST crosslinked with 40kDa PEG); (4) 20kDa PEG GLP1-Fc group (GLP-1-L1-Fc2-L2-ST crosslinked with 20kDa PEG); and (5) control group. The groups (1) to (4) were administered 3 mg/kg, and the control group was given normal saline. The IPGTT experiment was carried out at 24, 144, 168, 192, 216, and 240h after administration. The experimental procedure was as follows. The mice were fasted for 6h, and the blood glucose level and body weight were measured. During the fasting period, the mice have free access to water. After 6 hours, the mice were administered 2.0g/kg glucose solution with the volume of 10ml/kg intraperitoneally, and the blood glucose values at 10, 30, 60, 90, 120min after glucose load were measured. According to the values, the glucose tolerance curve was drawn and the area under the curve (AUC) of blood glucose was calculated. The data were represented as the mean $\pm$ standard error (Mean $\pm$SEM) and analyzed by Graphpad prism7.0 statistical software. The differences were analyzed with Mann-Whitney statistics.

As shown in figures 10A, 10B, 10C, 10D, 10E, and 10F, both the blood glucose and the area under the curve (AUC) in glucose tolerance test of each experimental group reduced significantly compared with those in the control group within 6 days after a single administration. After 7 days, there was no significant difference between the dulaglutide and GLP1-Fc groups and the control group, indicating that the efficacy of GLP1-Fc and dulaglutide can be maintained for 6 days. As shown in figures 10G, 10H, 10I, 10J, 10K and 10L, PEG (20kDa)-GLP1-Fc still had a significant glucose tolerance effect on the 8th day after a single administration. After 9 days, the area under the curve (AUC) of glucose tolerance test had no difference compared with the control group, indicating that the drug effect can be maintained for 8 days. PEG (40kDa)-GLP1-Fc still had a significant glucose tolerance effect until day 9 after a single administration. After 10 days, the area under the curve (AUC) of glucose tolerance test had no difference compared with the control group, indicating that the drug effect can be maintained for 9 days.

FIG. 10M shows the daily body weight changes of C57BL/6J mice after administration. Compared with the control group, the body weights of mice in each group decreased within 24 hours, especially in dulaglutide group and GLP1-Fc non-crosslinked with PEG group. It is worth noting that both PEG (20kDa)-GLP1-Fc and PEG (40kDa)-GLP1-Fc can significantly reduce the rapid body weight loss caused by drugs, indicating that PEGylation can significantly ameliorate the side effects of such drugs in clinical applications, and thus have better safety property.

(2) Experimental results of GLP1-L1-Fc4-L2-STG3

18 healthy male C57BL/6 mice aged 8-10 weeks were divided into 3 groups with 6 mice in each group. These groups include: (1) control group; (2) dulaglutide group; and (3) PEG (40kDa)-GLP1-Fc group (GLP1-L1-Fc4-L2-STG3 crosslinked with 40kDa PEG). Groups (2) and (3) were administered with equal activity (i.e., performing mass conversion based on *in vitro* activity detected in FIG. 19), wherein the dose of dulaglutide was 0.76 mg/kg, PEG (40kDa)-GLP1-Fc was 3.5 mg/kg, and the control group was given saline. The IPGTT experiment was carried out at 1, 5, 6, 7, and 8 days after administration, and the experimental procedure was as follows. The mice were fasted for 6h, and the blood glucose level and body weight were measured. During the fasting period, the mice have free access to water. After 6 hours, the mice were administered 2.0g/kg glucose solution with the volume of 10ml/kg intraperitoneally, and the blood glucose values at 10, 30, 60, 90, 120min after glucose load were measured. According to the values, the area under the curve (AUC) of blood glucose was calculated. The data were represented as the mean ± standard error (Mean ±SEM) and analyzed by Graphpad prism7.0 statistical software. The differences were analyzed with Mann-Whitney statistics.

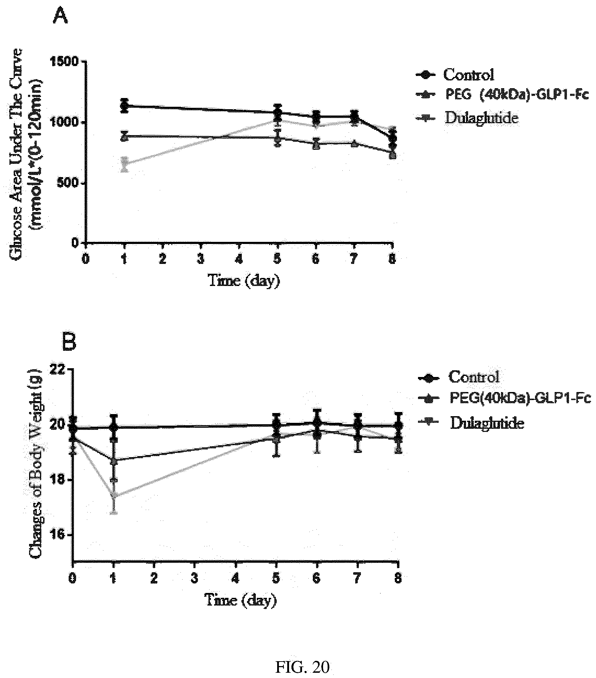

As shown in FIG. 20A, both the blood glucose and the area under the curve (AUC) of glucose tolerance test of mice in each experimental group reduced significantly compared with those in the control group within 5 days after a single administration. After 5 days, there was no significant difference between the dulaglutide and the control group, indicating that the efficacy of dulaglutide can be maintained for 5 days. PEG (40kDa)-GLP1-Fc still had a significant glucose tolerance effect until day 7 after a single administration. After 8 days, the area under the curve (AUC) of glucose tolerance test had no difference compared with the control group, indicating that the efficacy of PEG (40kDa)-GLP1-Fc can be maintained for 7 days.

FIG. 20B shows the body weight changes of C57BL/6 mice after administration. Compared with the control group, the body weight of mice in both groups decreased within 24 hours, especially in dulaglutide group. It is worth noting that PEG (40kDa)-GLP1-Fc can also significantly reduce the rapid body weight loss caused by drugs, which further illustrates that PEGylation can significantly ameliorate the side effects of such GLP-1 receptor agonist drugs in clinical applications .

Example 5 Detection of hypoglycemic activity of crosslinked GLP1-L1-Fc4-L2-STG3 in STZ-induced type II diabetic mice

Construction of type II diabetic mouse model

C57BL/6 mice (purchased from Beijing Vital River Co., Ltd.) were reared with 5 mice per cage, fed with normal mouse growth feed and free drinking water. The light time was 12h (08:00am-20:00pm) and the dark time was 12h (20:00pm-08:00am). 30 healthy female C57BL/6 mice aged 8-10 weeks were induced with 60mg/kg STZ, administered continuously for 4 days, and the blood glucose values were measured after 2 weeks. The mice with non-fasting blood glucose values ≥16.7mmol/L were considered as the successfully established type II diabetic mice.

Protocol

24 type II diabetic mice were divided into 4 groups with 6 mice in each group. These groups include: (1) control group; (2) dulaglutide-lmg/kg group; (3) PEG (40kDa)-GLP1-Fc (GLP1-L1-Fc4-L2-STG3 crosslinked with 40kDa PEG)-1mg/kg group; and (4) PEG (40kDa)-GLP1-Fc (GLP1-L1-Fc4-L2-STG3 crosslinked with 40kDa PEG)-3mg/kg group. The dosage of dulaglutide was 1 mg/kg, the dosage of PEG (40kDa)-GLP1-Fc was 1 mg/kg or 3 mg/kg, and the control group was given normal saline. Blood glucose values and body weights on days 0, 1, 2, 3, 4, 5, 6, 7, 8, 9, 10, 11, and 12 were measured with no fasting and free drinking. Data were represented as the mean ± standard error (Mean ±SEM) and analyzed by Graphpad prism7.0 statistical software. The differences were analyzed with Mann-Whitney statistics.

Results

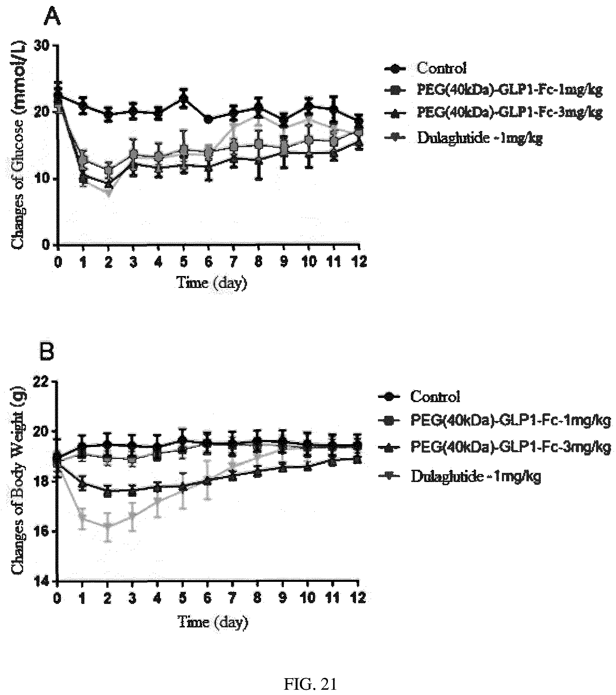

As shown in FIG. 21A, the blood glucose of mice in each experimental group reduced significantly compared with those in the control group after a single administration. The difference in blood glucose between the dulaglutide-lmg/kg group and the control group was still significant ($P$=0.0022) on day 6, but by day 7, there was no significant difference ($P$=0.9654), which indicated that the hypoglycemic effect of dulaglutide can be maintained for up to 6 days.

In the other hand, after a single administration, PEG (40kDa)-GLP1-Fc-1mg/kg still had a significant hypoglycemic effect until day 10 ($P$=0.0381), the result of which was not close to the control group until day 11 ($P$=0.1143); and PEG (40kDa)-GLP1-Fc-3mg/kg still had a significant hypoglycemic effect on day 11 after a single administration ($P$=0.0381), and the difference between which and the control group was not significant until day 12 ($P$=0.0762). The above results indicated that PEG (40kDa)-GLP1-Fc had a significantly better long-term hypoglycemic effect and can be used in the treatment of type II diabetes.

The body weight changes of diabetic mice after administration were further observed. As shown in FIG. 21B, the body weight of mice in all experimental groups decreased compared with those in the control group, especially in dulaglutide group, wherein the body weight of mice was reduced the most by 2.5g compared with the control group. The two dose groups of PEG (40kDa)-GLP1-Fc had a much smaller effect on body weight, wherein the body weight of mice in the PEG (40kDa)-GLP1-Fc-1mg/kg group was reduced by 0.3g at the most compared to the control group, and the body weight of the mice in the PEG (40kDa)-GLP1-3mg/kg group was reduced by 1.1g at the most compared to the control group. There results demonstrate that PEG (40kDa)-GLP1-Fc at various doses can significantly ameliorate the rapid body weight loss caused by drugs when used in the treatment of diabetes, indicating that PEGylation modifications can significantly ameliorate the side effects of such drugs in clinical applications, and effectively improve patient compliance at the same time.

In summary, PEGylated GLP1-Fc can promote insulin secretion by islet β-cells, inhibit the temporary increase in blood glucose caused by exogenous glucose intake, and promote glucose utilization. After PEG modification, GLP1-Fc protein shows longer enduring hypoglycemic effect, and fewer side effects, which avoid the rapid body weight loss often caused by GLP-1-like bodyweight-reducing drugs, and has a good clinical application prospect.

## Claims

1. A fusion protein of a GLP-1 receptor agonist, comprising a GLP-1 receptor agonist and a fusion partner (FP) capable of increasing the half-life of the fusion protein *in vivo,* wherein the GLP-1 receptor agonist and the fusion partner are linked directly or through a first linker L1;

   preferably, the first linker L1 is a peptide containing 1, 2, 3, 4, 5, 6, 7, 8, 9, 10, or more amino acids;

   more preferably, the first linker L1 is a flexible peptide containing A, T, G, and/or S, such as $(GS)_n$, where n is an integer from 1 to 50, for example, 1, 2, 3, 4, 5, 6, 7, 8, 9, or 10; and

   most preferably, the first linker L1 is selected from the group consisting of:

   GSGGGSGGGGSGGGGS (SEQ ID NO: 9),
   GSGGGGSGGGGSGGGGSGGGGSGGGGS (SEQ ID NO: 10),
   GGGGSGGGGSGGGGSGGGGS (SEQ ID NO: 11),
   GSGGGGSGGGGSGGGGSGGGGSGGGGSGGGGSGGGGS (SEQ ID NO: 12),
   GGGSGGGSGGGSGGGSGGGS (SEQ ID NO: 13),
   PRFQDSSSSKAPPPSLPSPSRLPGPSDTPILPQ (SEQ ID NO: 14),
   SSSSKAPPPSLPSPSRLPGPSDTPILPQ (SEQ ID NO: 15),
   SSSSKAPPPS (SEQ ID NO: 16),
   SRLPGPSDTPILPQ (SEQ ID NO: 17) and
   GGGGSGGGGSGGGGSA (SEQ ID NO: 18).

2. The fusion protein according to claim 1, wherein the GLP-1 receptor agonist is a full-length, truncated or variant form of GLP-1 or GLP-1 analogue, for example, Exendin-4, GLP -1(1-36), GLP-1(1-37), GLP-1(7-36), GLP-1(7-37), liraglutide, lixisenatide, albiglutide, dulaglutide, or semaglutide.

3. The fusion protein according to claim 1 or 2, wherein the fusion partner (FP) is a full-length, truncated or variant form of immunoglobulin Fc fragment, albumin, XTEN or transferrin;

   preferably, the Fc fragment is derived from a human immunoglobulin Fc fragment;

   preferably, the Fc fragment is composed of one to four domains selected from the group consisting of CH1 domain, CH2 domain, CH3 domain and CH4 domain;

   preferably, the Fc fragment is derived from the Fc fragment of IgG, IgA, IgD, IgE, or IgM, more preferably IgG Fc fragment;

   more preferably, the Fc fragment is derived from the Fc fragment of IgG1, IgG2, IgG3, or IgG4, and

   more preferably, the IgG Fc fragment has reduced ADCC effect and/or CDC effect and/or enhanced binding affinity to FcRn.

4. The fusion protein according to any one of claims 1 to 3, wherein the fusion partner (FP) is also linked directly or via a second linker L2 to an amino acid sequence ST containing a sortase recognition site, and wherein the sortase is, for example, sortase A or sortase B;

   preferably, the ST contains the core recognition site LPXTG of sortase A, wherein X is any amino acid, and the ST is, such as LPETG, LPETGG or LPETGGG;

   more preferably, the ST further contains an affinity tag linked to the sortase recognition site, and the ST is, for example, LPETGGHHHHHH or LPETGGWSHPQFEK; and

wherein the second linker L2 is a peptide containing 1, 2, 3, 4, 5, 6, 7, 8, 9, 10, or more amino acids;

more preferably, the second linker L2 is a flexible peptide containing A, T, G, and/or S, such as $(GS)_n$, where n is an integer from 1 to 50, for example, 1, 2, 3, 4, 5, 6, 7, 8, 9, or 10; and

most preferably, the second linker L2 is selected from the group consisting of:

GSGGGSGGGGSGGGGS (SEQ ID NO: 9),
GGGGSGGGGSGGGGSA (SEQ ID NO: 18),
GGGGS (SEQ ID NO: 19),
GGGGSGGGGS (SEQ ID NO: 20), and
GGGGSGGGGSGGGGS (SEQ ID NO: 21).

5. The fusion protein according to claim 4, having the structure of GLP-1-L1-FP-L2-ST, wherein GLP-1 represents the GLP-1 receptor agonist, and the GLP-1 receptor agonist, L1, L2, FP and ST have the same definition as in claim 4, and wherein either or both of L1 and L2 may not be present.

6. A conjugate, wherein one, two or more hydrophilic polymers are attached to the end of the fusion protein of any one of claims 1 to 5,

preferably, the hydrophilic polymer is attached to the sortase recognition site via a transpeptidation reaction,

in particular, the fusion protein may be in a form of monomer or dimer.

7. The conjugate according to claim 6, wherein the one, two or more hydrophilic polymers are each independently selected from polysaccharide and polyalkylene glycol, such as polypropylene glycol and polyethylene glycol; the polyalkylene glycol may be end-capped, for example, capped with alkoxy group such as methoxy group, and/or the polyalkylene glycol is linear or branched, and for example, the polyalkylene group is branched, such as branched polyethylene glycol, especially branched polyethylene glycol capped with methoxy group; the molecular weight of the polyalkylene glycol may be $\geq 1$, $\geq 10$, $\geq 20$, $\geq 30$, $\geq 40$, $\geq 50$, $\geq 60$, $\geq 70$, $\geq 80$, $\geq 90$, $\geq 100$, $\geq 110$, $\geq 120$, $\geq 130$, $\geq 140$, $\geq 150$, or $\geq 160$ kDa, for example, 5, 10, 20, 30, 40, 50, 60, 70, 80, 90, or 100 kDa, or a value between any two of the values; and preferably the molecular weight of the hydrophilic polymer is 20 kDa or 40 kDa.

8. A method for producing the conjugate of claim 6 or 7, comprising contacting the fusion protein of any one of claims 1 to 5 with sortase (such as sortase A or sortase B) and the hydrophilic polymer, wherein one end of the hydrophilic polymer has an amino group that can be amidated by sortase.

9. The method according to claim 8, wherein the one end of the hydrophilic polymer contains poly-Gly, such as GGGAA.

10. A pharmaceutical composition comprising an effective amount of the fusion protein of any one of claims 1 to 5 and/or the conjugate of claim 6 or 7, and optionally a pharmaceutically acceptable carrier.

11. The pharmaceutical composition according to claim 10 for use in reducing blood glucose or body weight, for example, in the treatment of diabetes, more specifically in the treatment of type I diabetes and/or type II diabetes, especially in the treatment of type II diabetes.

12. The pharmaceutical composition according to claim 10 or 11, wherein the pharmaceutical composition is in a form of liquid preparation or lyophilized preparation.

13. Use of the fusion protein of any one of claims 1 to 5 or the conjugate of claim 6 or 7 for the manufacture of a medicament for reducing blood glucose or body weight, wherein for example, the medicament is for use in treatment of diabetes, including type I diabetes and type II diabetes, especially type II diabetes.

14. A method for reducing blood glucose or body weight, comprising administering the fusion protein of any one of claims 1 to 5 or the conjugate of claim 6 or 7 to a subject in need thereof, for example, for treating diabetes, including type I diabetes and type II diabetes, especially type II diabetes.

15. A kit comprising the fusion protein of any one of claims 1 to 5, the conjugate of claim 6 or 7, and/or the pharmaceutical composition of any one of claims 10 to 12, and optional instructions for use.

FIG. 1

A

B

FIG. 2

## Glucose Metabolism

FIG. 3

## NH₄⁺ and Lac Metabolisms

FIG. 4

FIG. 5

FIG. 6

A

0min  30min  60min  90min

72KD

34KD

B

Double Cross-linking : 20.05%        Single Cross-linking : 55.57%

mAU

Time (min)

C

0min  30min  60min  90min

130KD

72KD

34KD

FIG. 7

**D**

Double Cross-linking : 3.79%    Single Cross-linking : 36.36%

**E**

Double Cross-linking : 12.37%    Single Cross-linking : 48.46%

FIG. 7 (continued)

A

B

PI  Single Cross-linking : 8.40%  Double Cross-linking : 90.84%  Substrate : 0.15%

C

P2  Single Cross-linking : 90.12%  Double Cross-linking : 8.56%  Substrate : 0.73%

FIG. 8

**Curve**

| y = ( (A - D)/(1 + (x/C)^B ) ) + D: | A | B | C | D | R^2 |
|---|---|---|---|---|---|
| ○ Dulaglutide (Standards: Concentration vs MeanVal... | 12276.58 | 1.249 | 0.24 | 2.78e6 | 1 |
| □ GLP1-Fc (Sample1: Concentration vs MeanValue) | 13124.39 | 1.26 | 0.241 | 2.76e6 | 1 |
| △ PEG (20kDa) -GLP1-Fc (Sample2: Concentration v... | 5954.553 | 1.191 | 0.601 | 2.81e6 | 0.999 |
| ◇ PEG (40kDa) -GLP1-Fc (Sample3: Concentration v... | 7790.08 | 1.179 | 0.821 | 3e6 | 0.999 |

| | EC50 | Relative Activity | Slope | Slope Ratio |
|---|---|---|---|---|
| Dulaglutide | 0.980 | / | 1.242 | / |
| GLP1-Fc | 0.999 | 98.10% | 1.254 | 1.01 |
| PEG ( 20kDa ) -GLP1-Fc | 2.427 | 40.38% | 1.183 | 0.95 |
| PEG ( 40kDa ) -GLP1-Fc | 2.983 | 32.85% | 1.173 | 0.94 |

FIG. 9

FIG. 10

FIG. 10 (continued)

E Glucose Tolerance Test -**168h**

F Glucose Area Under The Curve
**(mmol/L\*(0-120min)-168h**

FIG. 10 (continued)

G Glucose Tolerance Test -**192h**

Glucose Area Under The Curve
**(mmol/L*(0-120min)-192h**

H

FIG. 10 (continued)

FIG. 10 (continued)

FIG. 10 (continued)

EP 3 858 866 A1

FIG. 10 (continued)

FIG. 11

39

FIG. 12

FIG. 13

FIG. 14

FIG. 15

FIG. 16

FIG. 17

FIG. 18

FIG. 19

FIG. 20

FIG. 21

## INTERNATIONAL SEARCH REPORT

| International application No. |
|---|
| **PCT/CN2019/107709** |

**A. CLASSIFICATION OF SUBJECT MATTER**

C07K 19/00(2006.01)i; C12P 21/02(2006.01)i; A61K 38/26(2006.01)i; A61P 3/10(2006.01)i; A61P 3/04(2006.01)i

According to International Patent Classification (IPC) or to both national classification and IPC

**B. FIELDS SEARCHED**

Minimum documentation searched (classification system followed by classification symbols)

C07K; C12P; A61K; A61P

Documentation searched other than minimum documentation to the extent that such documents are included in the fields searched

Electronic data base consulted during the international search (name of data base and, where practicable, search terms used)

CNABS; CNKI; CNTXT; VEN; MEDLINE; EPTXT; USTXT; WOTXT and Keywords: 胰高血糖素样肽-1, GLP-1, Glucagon-like peptide-1, 杜拉鲁肽, dulaglutide, Fc, 融合, fus+ etc., 中国专利生物序列检索系统, Chinese Patent Biological Sequence Retrieval System; Genbank; EMBL和检索的序列: SEQ ID NO: 9-21 etc., EMBL and searched sequences: SEQ ID NO: 9-21 etc.

**C. DOCUMENTS CONSIDERED TO BE RELEVANT**

| Category* | Citation of document, with indication, where appropriate, of the relevant passages | Relevant to claim No. |
|---|---|---|
| X | WO 2018024162 A1 (SUNSHINE LAKE PHARMA CO., LTD.) 08 February 2018 (2018-02-08)<br>see embodiment 1, claims 1-14, sequences 1-11, and figures 1-10 | 1-3, 10-13, 15 |
| Y | WO 2018024162 A1 (SUNSHINE LAKE PHARMA CO., LTD.) 08 February 2018 (2018-02-08)<br>see embodiment 1, claims 1-14, sequences 1-11, and figures 1-10 | 4-9 |
| X | CN 101712722 A (LILLY CO. ELI) 26 May 2010 (2010-05-26)<br>see description, paragraphs [0009]-[0032], embodiment 1, and claims 1-18 | 1-3, 10-13, 15 |
| Y | CN 101712722 A (LILLY CO. ELI) 26 May 2010 (2010-05-26)<br>see description, paragraphs [0009]-[0032], embodiment 1, and claims 1-18 | 4-9 |
| Y | CN 104130308 A (JILIN UNIVERSITY) 05 November 2014 (2014-11-05)<br>see embodiment 2, claims 1-7, and sequences 2-8 | 4-9 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **23 December 2019** | **06 January 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2019/107709** |

| **Box No. II**      **Observations where certain claims were found unsearchable (Continuation of item 2 of first sheet)** |
| --- |

This international search report has not been established in respect of certain claims under Article 17(2)(a) for the following reasons:

1. ☑ Claims Nos.: **14**
    because they relate to subject matter not required to be searched by this Authority, namely:

    [1]   It relates to method of surgical or treatment performed on a human body or animal body and therefore do not warrant an international search by the international Searching Authority according to the criteria set out in PCT Rule 39.1(iv).

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2019/107709**

| Patent document cited in search report | | | Publication date (day/month/year) | Patent family member(s) | | | Publication date (day/month/year) |
|---|---|---|---|---|---|---|---|
| WO | 2018024162 | A1 | 08 February 2018 | CN | 107698684 | A | 16 February 2018 |
| CN | 101712722 | A | 26 May 2010 | NO | 20032565 | L | 01 August 2003 |
| | | | | JP | 2011006447 | A | 13 January 2011 |
| | | | | PT | 1355942 | E | 21 November 2008 |
| | | | | WO | 0246227 | A2 | 13 June 2002 |
| | | | | CY | 1108485 | T1 | 09 April 2014 |
| | | | | MX | PA03005036 | A | 05 September 2003 |
| | | | | NZ | 525577 | A | 27 May 2005 |
| | | | | HU | 229218 | B1 | 30 September 2013 |
| | | | | NO | 20032565 | A | 01 August 2003 |
| | | | | HU | 1300326 | A2 | 28 October 2003 |
| | | | | WO | 0246227 | A3 | 24 April 2003 |
| | | | | SK | 288342 | B6 | 01 March 2016 |
| | | | | CY | 1109377 | T1 | 02 July 2014 |
| | | | | IL | 155812 | D0 | 23 December 2003 |
| | | | | HU | 230603 | B1 | 28 March 2017 |
| | | | | ES | 2328510 | T3 | 13 November 2009 |
| | | | | SK | 6702003 | A3 | 03 August 2004 |
| | | | | NO | 20111369 | A | 01 August 2003 |
| | | | | EA | 200300644 | A1 | 25 December 2003 |
| | | | | EA | 005584 | B1 | 28 April 2005 |
| | | | | CA | 2716959 | A1 | 13 June 2002 |
| | | | | DE | 60135581 | D1 | 09 October 2008 |
| | | | | EP | 1724284 | A3 | 18 April 2007 |
| | | | | NO | 20111369 | L | 01 August 2003 |
| | | | | KR | 100942864 | B1 | 17 February 2010 |
| | | | | SI | 1355942 | T1 | 28 February 2009 |
| | | | | EP | 1724284 | A2 | 22 November 2006 |
| | | | | HU | 0302529 | A3 | 30 March 2009 |
| | | | | IL | 184429 | A | 15 April 2010 |
| | | | | PL | 209550 | B1 | 30 September 2011 |
| | | | | UA | 81897 | C2 | 25 February 2008 |
| | | | | ZA | 200303642 | A | 12 August 2004 |
| | | | | EC | SP064643 | A | 25 July 2009 |
| | | | | UA | 93662 | C2 | 10 March 2011 |
| | | | | CN | 1483041 | A | 17 March 2004 |
| | | | | SI | 1724284 | T1 | 31 December 2009 |
| | | | | KR | 20080085082 | A | 22 September 2008 |
| | | | | NO | 332221 | B1 | 30 July 2012 |
| | | | | PT | 1724284 | E | 30 September 2009 |
| | | | | IL | 184429 | D0 | 31 October 2007 |
| | | | | CZ | 20031602 | A3 | 12 April 2006 |
| | | | | EP | 1355942 | A2 | 29 October 2003 |
| | | | | ZA | 200303642 | B | 12 August 2004 |
| | | | | HU | 0302529 | A2 | 28 October 2003 |
| | | | | US | 2004053370 | A1 | 18 March 2004 |
| | | | | DE | 60139430 | D1 | 10 September 2009 |
| | | | | SK | 288088 | B6 | 03 June 2013 |
| | | | | AT | 437891 | T | 15 August 2009 |
| | | | | CA | 2434237 | A1 | 13 June 2002 |

Form PCT/ISA/210 (patent family annex) (January 2015)

**INTERNATIONAL SEARCH REPORT**
Information on patent family members

| | International application No. |
|---|---|
| | **PCT/CN2019/107709** |

| Patent document cited in search report | Publication date (day/month/year) | Patent family member(s) | Publication date (day/month/year) |
|---|---|---|---|
| CN 104130308 A | 05 November 2014 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201811125762
- US 477967 A
- US 478017 A
- US 478425 A
- US 5116964 A
- US 5541087 A
- WO 9734631 A
- WO 9632478 A
- WO 03040211 A
- US 6566506 B
- US 6864350 B
- US 6455639 B
- US 5932462 A
- EP 1245608 A

### Non-patent literature cited in the description

- *J Med Chem,* 2004, vol. 47, 4128-4134
- *Diabetes Care,* 1992, vol. 15, 270-276
- *Lancet,* 2002, vol. 359, 824-830
- *Endoer. Rev,* 1996, vol. 16, 390-410
- *Diabetologia,* 1985, vol. 28, 565-573
- *Diabetic Med,* 2001, vol. 18, 144-149
- *Diabetes,* 2002, vol. 51, 1443-1452
- *Diabetologia,* 2002, vol. 45, 1263-1273
- *Diabetes,* 2001, vol. 50, 525-529
- *Diabetes,* 2001, vol. 50, 725
- *Diabetes,* 2003, vol. 52, 365-371
- *Recent Prog. Hormne Res.,* 2001, vol. 56, 377-399
- *Disbetologia,* 1996, vol. 39, 1546-1553
- *Am. J. Physicl Endocrinol. Metab,* 2001, vol. 281, E242-247
- *Diabetes Care,* 1999, vol. 22, 403-408
- *J. Clin. Endocrinology and Metabolism,* 2003, vol. 88, 3082-3089
- *Diabetes,* 1995, vol. 44, 1295
- **CUNNINGHAM, B. ; WELLS, J.** *Science,* 1989, vol. 244, 1081-1085
- **SMITH, L. et al.** *J. Mol. Biol.,* 1992, vol. 224, 899-904
- **DE VOS, A. et al.** *Science,* 1992, vol. 255, 306-312
- **CAPON et al.** *Nature,* 1989, vol. 337, 525-531
- **CHAMOW et al.** *Trends Biotechnol.,* 1996, vol. 14, 52-60
- **H.NEURATH ; RL HILL.** The Proteins. Academic Press, 1979
- **BAILON et al.** Rational design of potent, long-lasting form of interferon: A 40 kDa branched polyethylene glycol-conjugated interferon $\alpha$-2a for the treatment of hepatitis C. *Bioconjugate Chem,* 2001, vol. 12, 195-202
- **BOWEN et al.** Relationship between molecular mass and duration of activity of polyethylene glycol conjugated granulocyte colony-stimulating factor mutein. *Experimental Hematology,* 1999, vol. 27, 425-32
- **BAILON et al.** PEG-modified biopharmaceuticals. *Expert Opin Deliv.,* 2009, vol. 6, 1-16
- Remington's Pharmaceutical Sciences. Mack Publishing Co,
- Sustained and Controlled Release Drug Delivery Systems. Marcel Dekker, Inc, 1978